(19) Europäisches Patentamt
European Patent Office
Office européen des brevets

(11) EP 3 950 947 A1

(12) EUROPEAN PATENT APPLICATION
published in accordance with Art. 153(4) EPC

(43) Date of publication:
**09.02.2022 Bulletin 2022/06**

(21) Application number: **20925416.8**

(22) Date of filing: **15.06.2020**

(51) Int Cl.:
***C12N 15/50*** *(2006.01)* ***C07K 14/165*** *(2006.01)*
***C12N 15/861*** *(2006.01)* ***A61K 39/215*** *(2006.01)*
***A61P 31/14*** *(2006.01)*

(86) International application number:
**PCT/CN2020/096024**

(87) International publication number:
**WO 2021/184560 (23.09.2021 Gazette 2021/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.03.2020 CN 202010193587**

(71) Applicants:
- **Academy of Military Medical Science, PLA**
  **Beijing 100071 (CN)**
- **Cansino Biologics Inc.**
  **Tianjin 300457 (CN)**

(72) Inventors:
- **CHEN, Wei**
  **Beijing 100071 (CN)**
- **WU, Shipo**
  **Beijing 100071 (CN)**
- **HOU, Lihua**
  **Beijing 100071 (CN)**
- **ZHANG, Zhe**
  **Beijing 100071 (CN)**
- **WANG, Busen**
  **Beijing 100071 (CN)**
- **GUO, Qiang**
  **Beijing 100071 (CN)**
- **ZHANG, Jinlong**
  **Beijing 100071 (CN)**
- **SONG, Xiaohong**
  **Beijing 100071 (CN)**
- **FU, Ling**
  **Beijing 100071 (CN)**
- **ZHANG, Jun**
  **Beijing 100071 (CN)**
- **CHEN, Yi**
  **Beijing 100071 (CN)**
- **ZHAO, Zhenghao**
  **Beijing 100071 (CN)**
- **ZHU, Tao**
  **Tianjin 300457 (CN)**
- **LI, Jin**
  **Tianjin 300457 (CN)**
- **SHEN, Chunlin**
  **Tianjin 300457 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) RECOMBINANT NOVEL CORONAVIRUS VACCINE USING REPLICATION-DEFICIENT HUMAN ADENOVIRUS AS VECTOR

(57) Provided is a novel coronavirus vaccine using replication-deficient human type 5 adenovirus as a vector. The vaccine takes the replication-deficient human type 5 adenovirus that is lack of E1 and E3 in a combined mode as a vector, and HEK293 cells that integrate adenovirus E1 genes serve as a packaging cell line, and protective antigenic genes carried are optimized COVID-19 (SARS-CoV-2) S protein genes (Ad5-nCoV). The vaccine has good immunogenicity in both mouse and guinea pig models and can induce the body to produce a strong cellular and humoral immune responses in a short time. Research on the protective effect of hACE2 transgenic mice shows that 14 days after a single Ad5-nCoV immunization, the viral load in lung tissues can be significantly reduced. It shows that the vaccine has a good immune protection effect against COVID-19.

EP 3 950 947 A1

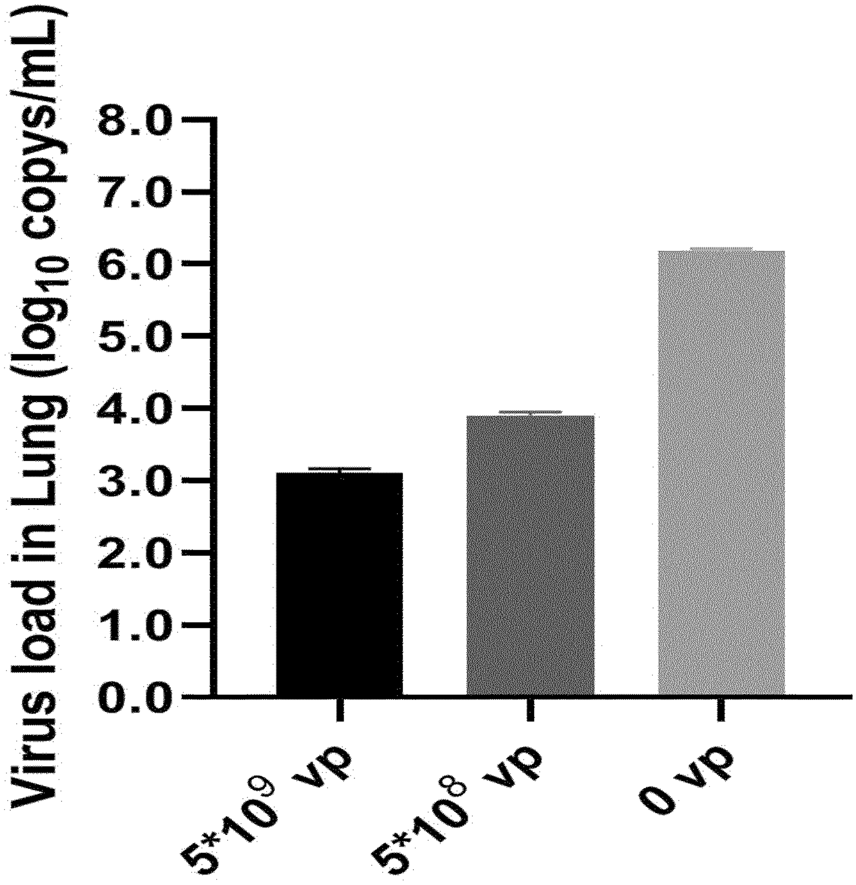
Virus load in Lung ($log_{10}$ copys/mL)
8.0
7.0
6.0
5.0
4.0
3.0
2.0
1.0
0.0
$5*10^9$ vp
$5*10^8$ vp
0 vp

FIG 16

<110>  ACADEMY OF MILITARY MEDICAL SCIENCE,PLA
CANSINO BIOLOGICS INC.

<120>  RECOMBINANT SARS-CoV-2 VACCINE USING HUMAN REPLICATION-DEFICIENT ADENOVIRUS AS VECTOR

<160>  2

<170>  SIPOSequenceListing 1.0

<210>  1
<211>  3876
<212>  DNA
<213>  Artificial Sequence

<400>  1
```
gaattcgccg ccaccatgga cgccatgaag cggggcctct gctgtgttct gctgctctgc      60
ggcgccgtgt tcgtgagtaa ctcgagccag tgcgtgaacc tgacaacaag gacacagctg     120
ccccctgcct acacaaacag cttcactagg ggcgtgtact accccgacaa ggtgttcagg     180
tccagcgtgc tgcacagcac acaggacctg ttcctgccct tcttcagcaa cgtgacatgg     240
ttccacgcca ttcacgtgag cgggaccaac gggaccaagc ggttcgataa ccctgtcttg     300
cccttcaacg atggcgtgta ctttgccagc accgagaagt ccaacatcat caggggctgg     360
atctttggca caaccctgga cagcaagacc cagagcctcc tgatcgtcaa caacgccaca     420
aacgtcgtga tcaaggtgtg cgagttccag ttctgcaacg atccattcct gggcgtgtac     480
taccataaga acaacaagtc ctggatggag agcgagttcc gggtctactc cagcgccaac     540
aactgcacct tcgagtacgt gagccagccc ttcctgatgg acttggaggg gaagcagggc     600
aacttcaaga acctccggga gttcgtcttt aagaacattg acggctactt caagatctac     660
tccaagcaca cccccatcaa cctcgtcagg gatctgcccc aggggtttag cgccctggag     720
cccctggtcg atctgccaat cggcatcaac atcacacggt ttcagaccct gctggccctg     780
caccggtcct acctcacccc tggcgatagc agctccggct ggacagccgg ggccgccgcc     840
tactacgtcg gctacctcca gcctcggact ttcctgctga agtacaacga gaacgggaca     900
atcaccgatg ccgtggactg cgccctggat cccctcagcg agaccaagtg cacactgaag     960
tcctttactg tggagaaggg gatctaccag acatccaact ttagggtgca gcccaccgag    1020
agcattgtca ggttccccaa catcacaaac ctgtgcccct ttggcgaggt gttcaacgcc    1080
acaagattcg cttccgtgta cgcctggaac aggaagcgga tcagcaactg cgtggccgat    1140
tactccgtcc tgtacaacag cgcctccttc tccaccttca agtgctacgg cgtgtccccc    1200
accaagctga acgatctgtg ctttactaac gtgtacgctg acagcttcgt gatcagaggc    1260
gatgaggtgc ggcagatcgc ccctgggcag acagggaaga tcgccgacta caactacaag    1320
ctgcccgatg acttcacagg gtgcgtgatc gcctggaact ccaacaacct cgatagcaag    1380
gtgggcggca actacaacta cctctacagg ctgtttagga agtccaacct gaagcccttt    1440
gagcgggata ttagcaccga gatctaccag gccgggagca ccccttgtaa cggcgtcgag    1500
gggtttaact gctactttcc tctgcagagc tacgggttcc agcccaccaa cggggtcggg    1560
taccagccat accgggtggt ggtgctgagc ttcgagctgc tgcacgcccc agccaccgtc    1620
tgcggcccca agaagtccac taacctggtg aagaacaagt gcgtgaactt caacttcaac    1680
ggcctgacag ggacaggcgt gctgacagag tccaacaaga agttcctccc cttccagcag    1740
tttgggcggg acattgccga cacaaccgat gccgtgcggg acccacagac cctggagatc    1800
ctggacatca caccctgcag cttcggcggg gtgagcgtga ttacacccgg cacaaacacc    1860
tccaaccagg tggccgtgct gtaccaggat gtgaactgca cagaggtccc cgtggccatt    1920
cacgccgatc agctgacccc cacctggcgg gtgtacagca ccggctccaa cgtgttccag    1980
actagggccg gctgcctgat cggggccgag cacgtgaaca acagctacga gtgcgacatc    2040
cccattgggg ccgggatctg cgcctcctac cagacacaga caaacagccc taggcgggcc    2100
aggtcggtgg ccagccagtc catcatcgcc tacaccatga gcctgggcgc cgagaacagc    2160
gtggcctaca gcaacaacag catcgctatc ccaacaaact ttaccatctc cgtgaccacc    2220
gagatcctgc ccgtcagcat gactaagaca tccgtcgact gcaccatgta catctgcggg    2280
gacagcaccg agtgctccaa cctgctgctg cagtacgggt ccttctgcac ccagctgaac    2340
agggccctga ctggcattgc cgtcgagcag gataagaaca cacaggaggt ctttgcccag    2400
```

```
gtgaagcaga tctacaagac acccccaatt aaggacttcg gcggcttcaa cttctcccag      2460
attctgcctg accccagcaa gcccagcaag cggtccttca tcgaggacct gctgttcaac      2520
aaggtgacac tggccgacgc cggctttatc aagcagtacg gcgactgcct cggcgacatc      2580
gccgctaggg acctgatctg cgcccagaag ttcaacggcc tgacagtgct gccccctctg      2640
ctgacagacg agatgatcgc ccagtacaca agcgccctgc tggccggcac catcacctcc      2700
gggtggacat tcggggccgg ggccgccctg cagatcccct ttgccatgca gatggcctac      2760
aggttcaacg gcattggcgt gacacagaac gtgctgtacg agaaccagaa gctgatcgcc      2820
aaccagttta actccgccat cgggaagatc caggattccc tgagcagcac cgccagcgcc      2880
ctgggcaagc tccaggatgt ggtgaaccag aacgcccagg ccctcaacac cctggtgaag      2940
cagctgtcct ccaacttcgg cgccattagc tccgtgctga acgacatcct gagccggctg      3000
gacaaggtgg aggccgaggt gcagattgac cggctgatta ccggacggct gcagtccctg      3060
cagacctacg tgacacagca gctcatccgg gccgccgaga tccgcgcctc cgccaacctg      3120
gccgccacta agatgtccga gtgcgtgctc ggccagagca agagggtgga tttctgcggg      3180
aagggctacc acctgatgag cttcccccag agcgcccccc atggggtggt gttcctgcac      3240
gtgacatacg tgcctgccca ggagaagaac ttcaccaccg ccccagccat ttgccacgac      3300
ggcaaggccc acttccctag ggagggcgtg ttcgtgagca acgggacaca ctggttcgtg      3360
acccagcgga acttctacga gccccagatt atcaccacag ataacacctt tgtgtccggg      3420
aactgcgatg tcgtgattgg gatcgtcaac aacacagtct acgacccccct gcagcccgag     3480
ctcgatagct ttaaggagga gctggataag tactttaaga accacacctc cctgatgtg       3540
gacctggggg atatcagcgg catcaacgcc agcgtggtga acatccagaa ggagatcgat      3600
aggctgaacg aggtggccaa gaacctgaac gagtccctga tcgacctgca ggagctgggg      3660
aagtacgagc agtacatcaa gtggccctgg tacatctggc tgggcttcat cgccgggctg      3720
atcgccatcg tgatggtgac cattatgctc tgctgcatga ctagctgctg ctcctgcctg      3780
aaggggtgct gcagctgcgg gagctgctgc aagtttgatg aggatgatag cgagccagtg      3840
ctgaagggcg tgaagctgca ctacacctga aagctt                                3876
```

<210> 2
<211> 3843
<212> DNA
<213> SARS-CoV-2

<400> 2

```
cccgggggccg ccaccatgtt tgtttttctt gttttattgc cactagtctc tagtcagtgt      60
gttaatctta caaccagaac tcaattaccc cctgcataca ctaattcttt cacacgtggt     120
gtttattacc ctgacaaagt tttcagatcc tcagttttac attcaactca ggacttgttc     180
ttacctttct tttccaatgt tacttggttc catgctatac atgtctctgg gaccaatggt     240
actaagaggt ttgataaccc tgtcctacca tttaatgatg gtgtttattt tgcttccact     300
gagaagtcta acataataag aggctggatt tttggtacta ctttagattc gaagacccag     360
tccctactta ttgttaataa cgctactaat gttgttatta aagtctgtga atttcaattt     420
tgtaatgatc catttttggg tgtttattac cacaaaaaca acaaaagttg gatggaaagt     480
gagttcagag tttattctag tgcgaataat tgcacttttg aatatgtctc tcagcctttt     540
cttatggacc ttgaaggaaa acagggtaat ttcaaaaatc ttagggaatt tgtgtttaag     600
aatattgatg gttattttaa aatatattct aagcacacgc ctattaattt agtgcgtgat     660
ctccctcagg gtttttcggc tttagaacca ttggtagatt tgccaatagg tattaacatc     720
actaggtttc aaactttact tgctttacat agaagttatt tgactcctgg tgattcttct     780
tcaggttgga cagctggtgc tgcagcttat tatgtgggtt atcttcaacc taggactttt     840
ctattaaaat ataatgaaaa tggaaccatt acagatgctg tagactgtgc acttgaccct     900
ctctcagaaa caaagtgtac gttgaaatcc ttcactgtag aaaaaggaat ctatcaaact     960
tctaacttta gagtccaacc aacagaatct attgttagat ttcctaatat tacaaacttg    1020
tgcccttttg gtgaagtttt taacgccacc agatttgcat ctgtttatgc ttggaacagg    1080
aagagaatca gcaactgtgt tgctgattat tctgtcctat ataattccgc atcattttcc    1140
acttttaagt gttatggagt gtctcctact aaattaaatg atctctgctt tactaatgtc    1200
tatgcagatt catttgtaat tagaggtgat gaagtcagac aaatcgctcc agggcaaact    1260
ggaaagattg ctgattataa ttataaatta ccagatgatt ttacaggctg cgttatagct    1320
tggaattcta acaatcttga ttctaaggtt ggtggtaatt ataattacct gtatagattg    1380
tttaggaagt ctaatctcaa accttttgag agagatattt caactgaaat ctatcaggcc    1440
ggtagcacac cttgtaatgg tgttgaaggt tttaattgtt actttccttt acaatcatat    1500
ggtttccaac ccactaatgg tgttggttac caaccataca gagtagtagt actttctttt    1560
```

```
gaacttctac atgcaccagc aactgtttgt ggacctaaaa agtctactaa tttggttaaa     1620
aacaaatgtg tcaatttcaa cttcaatggt ttaacaggca caggtgttct tactgagtct     1680
aacaaaaagt ttctgccttt ccaacaattt ggcagagaca ttgctgacac tactgatgct     1740
gtccgtgatc cacagacact tgagattctt gacattacac catgttcttt tggtggtgtc     1800
agtgttataa caccaggaac aaatacttct aaccaggttg ctgttcttta tcaggatgtt     1860
aactgcacag aagtccctgt tgctattcat gcagatcaac ttactcctac ttggcgtgtt     1920
tattctacag gttctaatgt ttttcaaaca cgtgcaggct gtttaatagg ggctgaacat     1980
gtcaacaact catatgagtg tgacataccc attggtgcag gtatatgcgc tagttatcag     2040
actcagacta attctcctcg gcgggcacgt agtgtagcta gtcaatccat cattgcctac     2100
actatgtcac ttggtgcaga aaattcagtt gcttactcta ataactctat tgccataccc     2160
acaaatttta ctattagtgt taccacagaa attctaccag tgtctatgac caagacatca     2220
gtagattgta caatgtacat ttgtggtgat tcaactgaat gcagcaatct tttgttgcaa     2280
tatggcagtt tttgtacaca attaaaccgt gctttaactg gaatagctgt tgaacaagac     2340
aaaaacaccc aagaagtttt tgcacaagtc aaacaaattt acaaaacacc accaattaaa     2400
gattttggtg gttttaattt ttcacaaata ttaccagatc catcaaaacc aagcaagagg     2460
tcatttattg aagatctact tttcaacaaa gtgacacttg cagatgctgg cttcatcaaa     2520
caatatggtg attgccttgg tgatattgct gctagagacc tcatttgtgc acaaaagttt     2580
aacggcctta ctgttttgcc acctttgctc acagatgaaa tgattgctca atacacttct     2640
gcactgttag cgggtacaat cacttctggt tggacctttg gtgcaggtgc tgcattacaa     2700
ataccatttg ctatgcaaat ggcttatagg tttaatggta ttggagttac acagaatgtt     2760
ctctatgaga accaaaaatt gattgccaac caatttaata gtgctattgg caaaattcaa     2820
gactcacttt cttccacagc aagtgcactt ggaaaacttc aagatgtggt caaccaaaat     2880
gcacaagctt taaacacgct tgttaaacaa cttagctcca attttggtgc aatttcaagt     2940
gttttaaatg atatcctttc acgtcttgac aaagttgagg ctgaagtgca aattgatagg     3000
ttgatcacag gcagacttca aagtttgcag acatatgtga ctcaacaatt aattagagct     3060
gcagaaatca gagcttctgc taatcttgct gctactaaaa tgtcagagtg tgtacttgga     3120
caatcaaaaa gagttgattt ttgtggaaag ggctatcatc ttatgtcctt ccctcagtca     3180
gcacctcatg gtgtagtctt cttgcatgtg acttatgtcc ctgcacaaga aaagaacttc     3240
acaactgctc ctgccatttg tcatgatgga aaagcacact ttcctcgtga aggtgtcttt     3300
gtttcaaatg gcacacactg gtttgtaaca caaaggaatt tttatgaacc acaaatcatt     3360
actacagaca acacatttgt gtctggtaac tgtgatgttg taataggaat tgtcaacaac     3420
acagtttatg atcctttgca acctgaatta gactcattca aggaggagtt agataaatat     3480
tttaagaatc atacatcacc agatgttgat ttaggtgaca tctctggcat taatgcttca     3540
gttgtaaaca ttcaaaaaga aattgaccgc ctcaatgagg ttgccaagaa tttaaatgaa     3600
tctctcatcg atctccaaga acttggaaag tatgagcagt atataaaatg gccatggtac     3660
atttggctag gttttatagc tggcttgatt gccatagtaa tggtgacaat tatgctttgc     3720
tgtatgacca gttgctgtag ttgtctcaag ggctgttgtt cttgtggatc ctgctgcaaa     3780
tttgatgaag acgactctga gccagtgctc aaaggagtca aattacatta cacataagtc     3840
gac                                                                   3843
```

## Description

### TECHNICAL FIELD

The present invention relates to a recombinant novel coronavirus vaccine for preventing the novel coronavirus epidemics. The present invention belongs to the field of bioengineering technology

### BACKGROUND OF THE PRESENT INVENTION

The 2019 novel coronavirus, that is, SARS-CoV-2 (also known as 2019-nCoV), was named by the World Health Organization on January 12, 2020. SARS-CoV-2 is a new strain of coronavirus that has never been previously found in the human body, which is the seventh member of coronavirus (CoV) that can infect humans. The incubation period of 2019-nCoV after infected is generally 1 to 14 days, and the common signs after infected include respiratory symptoms, fever, cough, shortness of breath and dyspnea. In severe cases, infection may lead to pneumonia, severe acute respiratory syndrome, renal failure, and even death. As of March 17, 2020, 81116 cases with confirmed diagnosis and 3231 deaths have been reported in China; and 98,486 cases and 3,848 cumulative deaths have been reported in the rest of the world, with an average mortality rate of 3.94%.

The novel coronavirus belongs to the Beta coronavirus genus, wrapped with an envelope, having round or elliptical, usually pleomorphic particles, with a diameter of 60 - 140 nm. Phylogenetic analysis of the whole genome of the virus (29,903 nucleotides) indicates that the coronavirus is most closely related to (89.1% nucleotide similarity) a group of SARS-like coronaviruses previously found in bats in China (genus Betacoronavirus, subgenus Sarbecovirus). In the evolutionary tree of S gene, 2019-nCoV is most closely related to the bat coronavirus bat-SL-CoVZC45, with an amino acid identity of 82.3%, while 2019-nCoV and SARS-CoV share approximately 77.2% amino acid identity. When 2019-nCoV entering human cell, the receptor that binds to 2019-nCoV is angiotensin converting enzyme 2 (ACE2), which is the same as that binds to SARS-CoV.

### TECHNICAL PROBLEM

Coronaviruses are a large family of viruses. So far, SARS-CoV, MERS-CoV and SARS-CoV-2 are the main causes of human infection outbreaks. There are few reports on the structure, function, key targets of infection, mechanism of action, and vaccine development of SARS-CoV-2. Several studies mainly focus on SARS-CoV and MERS-CoV vaccines, with main types of research and development including viral vector vaccines, nucleic acid vaccines, subunit vaccines, virus-like particle vaccines, inactivated vaccines, and live attenuated vaccines. The target antigen of the recombinant coronavirus vaccine is generally in the forms of full-length S protein and truncated S protein (S1 and RBD). The types of novel coronavirus vaccines under development include adenovirus vector vaccines, mRNA vaccines, DNA vaccines, recombinant protein vaccines and inactivated vaccines, and the results have not been reported yet. The purpose of the present invention is to provide a recombinant novel coronavirus vaccine.

### SUMMARY OF THE PRESENT INVENTION

For the above purposes, the present invention firstly provides an optimized polynucleotide whose sequence as shown in SEQ ID NO: 1 for encoding the S protein of the novel coronavirus used as vaccine. The recombinant adenovirus-vectored novel coronavirus vaccine is obtained after the said polynucleotide is packaged via a replication-deficient human type-5 adenovirus with deletion of E1 and E3 as a vector, and HEK293 cell integrated with adenovirus E1 gene as a packaging cell line.

The present invention also provides a vector containing the above polynucleotide.

In a preferred embodiment, the vector is pDC316.

The present invention also provides a human replication-deficient recombinant adenovirus expressing the above polynucleotide.

In a preferred embodiment, the recombinant human adenovirus is derived from the AdMax adenovirus system.

The present invention also provides the use of the above-mentioned recombinant adenovirus in preparation of the vaccine for preventing novel coronavirus disease.

In a preferred embodiment, the recombinant adenovirus is prepared as an injection, nasal drops or spray in the above use.

In a more preferred embodiment, the recombinant adenovirus is prepared as an intramuscular injection.

Finally, the present invention provides a method for preparing the above-mentioned recombinant adenovirus expressing the S protein of the novel coronavirus. The method includes the following steps:

(1) Construction of a shuttle plasmid vector containing the polynucleotide encoding the S protein of the 2019 novel coronavirus;
(2) Co-transfection of the shuttle plasmid vector of step (1) and backbone plasmid into a host cell;
(3) Cultivation of the host cells of step (2);
(4) Harvest of the human replication-deficient recombinant adenovirus released from the cells of step (3);
(5) Enlarge cultivation of the recombinant adenovirus of step (4);
(6) Purification of the culture product of step (5).

Preferably, the shuttle plasmid vector of step (1) is pDC316.

Preferably, the backbone plasmid of step (2) is pBHGlox∆E1,3Cre. Both plasmids belong to the AdMax adenovirus system, and are used together in host cell for packaging of recombinant adenovirus containing the above-mentioned polynucleotide encoding the S protein of the novel coronavirus.

Preferably, the cell of step (3) is HEK293 cells.

Preferably, the enlarge cultivation method of step (5) is suspension culture.

Preferably, the purification method of step (6) is Source 30 Q chromatography.

BENEFICIAL EFFECTS OF THE PRESENT INVENTION

When applied in mouse and guinea pig models as the novel coronavirus vaccine (Ad5-nCoV), the recombinant adenovirus expressing the S protein of the novel coronavirus provided by the present invention has shown good immunogenicity, and can induce strong cellular and humoral immune response in a short time. The protective effect on hACE2 transgenic mice suggests that a single dose of Ad5-nCoV could significantly reduce the viral load in the lung tissue on Day 14 after immunization, indicating that the vaccine has a favorable immune protection effect against the 2019 novel coronavirus. The preparation of the vaccine is quick and easy, which can be produced on a large scale in a short period of time to respond to sudden outbreaks.

BRIEF DESCRIPTION OF THE DRAWINGS

Figure 1 provides the map of shuttle plasmid pDC316-nCoV_Sopt;
Figure 2 is the identification map of S protein expressed in cells;
Figure 3 depicts the Western blot identification map of the expressed target antigen of the primary virus strain being the recombinant novel coronavirus vaccine candidate;
Figure 4 is the comparison of serum IgG antibody levels in mice on Day 9 after immunized with Ad5-nCoV intramuscularly;
Figure 5 shows the comparison of serum IgG antibody levels in mice on Day 9 and Day 14 after immunized with Ad5-nCoV intramuscularly;
Figure 6 provides the comparison of serum IgG antibody levels in mice immunized intramuscularly by differently constructed of recombinant adenoviruses;
Figure 7 is the comparison of serum antibody levels in mice immunized by different routes on Day 14 after vaccination with recombinant adenovirus;
Figure 8 depicts the comparison of $CD8^+$ T cell immune response induced by Ad5-nCoV;
Figure 9 shows the comparison of $CD4^+$ T cell immune response induced by Ad5-nCoV;
Figure 10 is the representative graph of cellular immune response in the intramuscular injection group;
Figure 11 provides the representative graph of cellular immune response in the intranasal immunization group;
Figure 12 shows the representative graph of cellular immune response in the control group;
Figure 13 depicts the comparison of antibody levels in trachea-lung washes on Day 14 after immunization with Ad5-nCoV;
Figure 14 is the comparison of the immunogenicity of Ad5-nCoV in guinea pig model;
Figure 15 shows the comparison of the percentage of weight loss in mice after SARS-CoV-2 challenge;
Figure 16 provides the comparison of viral load in lung tissue of mice after SARS-CoV-2 challenge.

EXAMPLES

The present invention is furtherly described with specific examples below, and the advantages and features of the present invention will become clearer with the description. However, these examples are only exemplary, and do not constitute any limitations on the protection scope defined by the claims of the present invention.

Example 1. Preparation of recombinant novel coronavirus vaccine using human replication-deficient adenovirus as vector.

1. Optimization and synthesis of S protein gene

The target antigen of the recombinant novel coronavirus vaccine is the S protein of the novel coronavirus strain (Genebank number: NC_045512.2). The expression level of the S protein is increased through optimization of the S protein gene, thereby improving the immunogenicity of the vaccine.

Firstly, codon optimization is conducted by UpGene software (Gao, W. Rzewski, A. Sun, H. Robbins, P. D. &Gambotto, A. UpGene: Application of a web-based DNA codon optimization algorithm. Biotechnol Prog, 2004. 20 ( 2 ) : p. 443-8.), to change most of the rare codons of the S protein gene to high-frequency codons. Then, considering that optimization through software may mechanically change the codons to those used at highest frequency, the protein translation efficiency may not be significantly improved due to the influence of the use efficiency of tRNA, mRNA secondary structure and other factors. In this case, manual analysis is used to replace part of the high-frequency and low-frequency codons, while the high-frequency and low-frequency codons are uniformly distributed in the S protein gene. Meanwhile, considering that increase the content of GC in mRNA may help to enhance the stability of mRNA, the content of GC in the S protein gene is appropriately increased, and G, C nucleotides are distributed in the entire S gene as evenly as possible. Also, the original signal peptide (1aa-12aa) of the S protein is changed to the tPA, i.e, tissue plasminogen activator signal peptide, to furtherly enhance the expression level of the S protein.

Before gene optimization, the content of rare codons (use frequency <70%) in the novel coronavirus S protein gene is 34%, high-frequency codons (use frequency> 90%) is 23%, and GC is 36%. The optimized gene shares 70.4% of homology with the original S protein gene sequence. The content of rare codons drops to 3%, the content of high-frequency codons increases to 81%, and the content of GC increases to 58%.

After the optimization of S protein gene, Kozak sequence is added ahead of the initiation codon, and restriction enzyme EcoRI is inserted at the upstream of the entire sequence, and HindIII is inserted at the downstream. The gene sequence is synthesized. Meanwhile, the original sequence of the S protein gene is synthesized as a control. The sequence of S protein gene with optimization (EcoRI and HindIII as the restriction enzyme) is shown in SEQ ID NO: 1, and the original sequence of S protein gene (SmaI and SalI as the restriction enzyme) is shown in SEQ ID NO: 2.

2. Construction of the vectors and identification the expression of S protein in vitro

2.1 Construction of the vectors

The above synthesized gene sequence is double-digested with EcoRI and HindIII, or SmaI and SalI, and the target gene fragment is extracted and connected to the shuttle plasmid pDC316 of the AdMax adenovirus system (Microbix Biosystems Inc., Canada), and then is transformed into DH5-$\alpha$ competent cells and spread on $Amp^r$ LB plate. Monoclones are selected for PCR identification, and sequencing verification is performed for those positive clones. Plasmid containing S protein gene sequence without optimization is marked as pDC316-nCoV_S, whereas plasmid containing S protein gene with optimization is marked as pDC316-nCoV_Sopt. The plasmid map of pDC316-nCoV_Sopt is shown in Figure 1.

Meanwhile, the optimized S protein gene sequence encoding the original signal peptide is amplified by overlap extension PCR, and is connected to the pDC316 vector, which is marked as pDC316-nCoV_oriSIP-Sopt; the non-optimized S protein gene sequence leading by tPA signal peptide is amplified and connected to the pDC316 vector, which is marked as pDC316-nCoV _tPA-S; the optimized gene sequence encoding S1 protein (S1 protein is the truncated S protein at amino acids 12-685) leading by tPA signal peptide (corresponding gene sequence with optimization is 25bp to 2103bp of SEQ ID NO: 1) is connected to the pDC316 vector, which is marked as pDC316-nCoV_S1opt; and the original gene sequence encoding S1 protein leading by tPA signal peptide is connected to the pDC316 vector, which is marked as pDC316-nCoV_S1.

2.2 Identification of the expression of S protein in vitro

The 6 shuttle plasmids constructed above and the pDC316 vector are transfected into HEK293 cells by TurboFect Transfection Reagent (Thermo Scientific, REF, R0531), and the cells are collected 48 hours later for Western blot. The method is as follows:
Transfection : On the day before the experiment, HEK293 cells are inoculated into a 6-well plate with $8\times10^5$ cells/well and cultured overnight at 37 °C in 5% $CO_2$ incubator. One hour prior to transfection, the medium is replaced with fresh DMEM medium containing 2% FBS, with 2 mL per well. During transfection, 2 $\mu$g of each plasmid is added to each 200 $\mu$L of FBS-free DMEM medium, mixed, and 3 $\mu$L of transfection reagent is added and mixed gently. The mixture is incubated at room temperature for 15 minutes, and gently dripped into the wells of the 6-well plate and mixed by gently shaking the plate. The plates are cultured at 37 °C in 5% $CO_2$ incubator. 5 hours later, the medium is replaced with fresh DMEM medium containing 10% FBS. Cells are collected 48 hours later to prepare samples for Western blot.

Sample preparation: 48 hours after transfection, the medium is carefully aspirated and discarded, cells are resuspended in PBS and centrifuged at 500 g for 5 minutes, and the supernatant is discarded. Cells are resuspended with 200 μL of RIPA buffer (THERMO Scientific, Prod # 89900, supplemented with appropriate amount of protease inhibitors and nucleases), placed on ice for 15 minutes, and centrifuged at 12000 rpm for 5 minutes at 4 °C. The supernatant is collected, and 1/3 volume of 4×SDS-PAGE loading buffer containing 200 mmol/L DTT is added. The supernatant is heated at 95 °C for 5 minutes, and stored frozen for Western blot.

Western blot: 10-well 4%-20% SDS-PAGE gradient gel is used for SDS-PAGE, and the loading quantity of each sample is 30 μL per well. Electrophoresis conditions: 80 V, 15min; 180V, until the bromophenol blue migrates out of the gel. The protein on the SDS-PAGE gel is transferred to nitrocellulose membrane by an electric rotating apparatus at 300 mA for 1 hour. After electroporation, the nitrocellulose membrane is blocked with 5% skim milk for 1 hour, and then the anti-S-protein rabbit polyclonal antibody (Sino Biological, Cat#40150-T52) at a dilution of 1: 2000 is added, and incubates at 4 °C overnight. The membrane is washed 4 times with washing buffer and shaken on the shaker for 5 minutes each time. Then HRP-labeled goat anti-rabbit IgG antibody (CST, Cat#7074S) at a dilution of 1:10000 in 5% skim milk is added. The membrane is incubated for 1 hour at room temperature, and washed 4 times with washing buffer. Immobilon™ Western Chemiluminescent HRP Substrate (MILLIPORE, Cat#WBKLS0500) is used for chemiluminescence reaction, and a chemiluminescence imager is used to capture images with different exposure times.

β-actin is used as the internal reference, and results are shown in Figure 2, wherein lane 1 indicates the cell transfected with pDC316-nCoV_S ; lane 2 depicts the cell transfected with pDC316-nCoV _tPA-S; lane 3 represents the cell transfected with pDC316-nCoV_oriSIP-Sopt; lane 4 shows the cell transfected with pDC316-nCoV_Sopt; lane 5 demonstrates the cell transfected with empty pDC316 vector; lane 6 indicates the prestained protein molecular weight marker; lane 7 depicts the cell transfected with empty pDC316 vector; lane 8 represents the cell transfected with pDC316-nCoV_S1; and lane 9 shows the cell transfected with pDC316-nCoV_S1opt. The results suggest that no expression of the associated protein is detected in the plasmid-transfected cells with the full-length S gene and S1 gene which without gene optimization; the S protein can be detected in the transfected cells after gene optimization, and the expression level is furtherly improved after replacing the original signal peptide by tPA; meanwhile, significant expression of S1 protein can also be detected after gene optimization.

3. Packaging, preparation and identification of recombinant adenovirus

3.1 Packaging of the recombinant adenovirus

The above constructed vectors pDC316-nCoV_Sopt, pDC316-nCoV_oriSIP-Sopt and pDC316-nCoV_S1opt are respectively co-transfected with the backbone plasmid pBHGloxΔE1,3Cre of the AdMax adenovirus system into HEK293 cells to package the recombinant adenovirus. The process is as follows:

a) On the day before transfection, HEK293 cells are inoculated into a 6-well plate with $8 \times 10^5$ cells/well, with MEM + 10% FBS as the medium, and are cultured overnight at 37 °C in 5% $CO_2$ incubator.
b) On the day of transfection, the medium is replaced, and the cells continue to be cultured in fresh MEM medium containing 10% FBS. When cells grow to cover 80%-90% of the bottom area, the backbone plasmid (pBHGloxΔE1, 3Cre) and shuttle plasmid are taken to transfect with TurboFect transfection reagent (Thermo scientific, REF, R0531) according to the manufacturer's instructions. The specific steps are as follows:

(1) 3.2 μg of backbone plasmid and 0.8 μg of shuttle plasmid are needed per transfection well, and mixed. The plasmids are diluted with 400 μL Opti-MEM medium.
(2) 6 μL of TurboFect transfection reagent is taken to added into the plasmids diluent in Opti-MEM medium, and gently mixed.
(3) The mixture of transfection reagent and plasmids are left at room temperature for 20 minutes, and then added to the cells.

c) On the day following transfection, the cells which cover the whole bottom are passaged into a 25 $cm^2$ cell culture flask, and continue to be cultured in MEM medium containing 5% FBS. Daily observation is conducted, and cells are passaged into a 75 $cm^2$ cell culture flask when they cover the bottom of the flask. Daily observation for signs of virus generation is performed. The phenomenon of virus generation is that the cells become large and round, in shape of grape, and obvious plaques begin to appear. Virus are collected when most of cells are diseased and detached from the bottom.

d) Cells with virus generation are resuspended, and centrifugated at 500 g for 10 minutes. The supernatant is discarded, and cells are resuspended in 2 mL PBS, and frozen in a refrigerator at -70 °C or lower and thawed in a water bath at 37 °C for three times. Cells are centrifugated at 12000 g for 10 minutes, the supernatant containing viruses is collected, and the precipitate is discarded.

The virus strain containing the optimized full-length S protein gene sequence leading by tPA signal peptide is marked as Ad5-nCoV_Sopt, the strain containing the optimized full-length S protein gene sequence leading by the original signal peptide is marked as Ad5-nCoV_oriSIP-Sopt; and the strain containing tPA signal peptide plusing the optimized S1 protein gene sequence is marked as Ad5-nCoV_S1opt.

3.2 Identification of recombinant adenovirus

3.2.1 PCR amplification of S and S1 gene and sequencing

The following universal primers of pDC316 vector are used to amplify the complete sequence of S or S1 protein:

pDC316-F: ACGTGGGTATAAGAGGCG
pDC316-R: CGATGCTAGACGATCCAG

2 μL of proteinase K is added to 50 μL of vaccine candidate solution, and digested at 50 °C for 30 minutes to release the genome of the virus, which is used as the template to amplify the gene sequences of S protein or S1 protein.

PCR amplification system:

| | |
|---|---|
| Vaccine candidate genome | 1.0 μL |
| Upstream primer | 1.0 μL |
| Downstream primer | 1.0 μL |
| dNTP Mix | 4.0 μL |
| Pyrobest DNA Polymerase | 0.5μL |
| 10×Buffer | 5.0 μL |
| $ddH_2O$ | 37.5 μL |

Reaction procedure:

| | | |
|---|---|---|
| 94°C | 5 min | |
| 94°C | 30 s | 30 cycles |
| 56°C | 30 s | |
| 72°C | 190 s (or S1 gene, 120s) | |
| 72°C | 10 min | |

The results of agarose gel electrophoresis show that a single target band could be amplified in all three candidates with correct fragment size. The target bands are extracted and sequenced, and the alignment results indicate that the sequences tested are completely correct.

3.2.2 Identification the expression of target antigen

HEK293 cells are infected with the vaccine candidates constructed above, and are collected 48 hours later for Western blot detection of the target antigen. Significant expression of the target protein can be detected in all the three candidates, Ad5-nCoV_Sopt, Ad5-nCoV_oriSIP-Sopt and Ad5-nCoV_S1opt, as shown in Figure 3, wherein lane 1 indicates the prestained protein molecular weight marker; lane 2 and 3 depict Ad5-nCoV_oriSIP-Sopt infected cells; lane 4 and 5 represent Ad5-nCoV_Sopt infected cells; lane 6 indicates Ad5-nCoV_S1opt infected cells; and lane 7 and 8 depict the uninfected cells.

3.2.3 Culture of the recombinant adenovirus

HEK293 cells are suspension-cultured at 37 °C in 5% $CO_2$, at 130 rpm. When inoculated with the virus strain, cells with a viability greater than 95% are diluted to $1.0 \times 10^6$ cells/mL, with 300 mL as the final volume. HEK293 cells are infected with the fifth generation of the recombinant adenovirus at MOI 10, and are cultured at 37 °C in 5% $CO_2$, with shaken at 130 rpm. Samples are collected every 24 hours for the measurement of cell viability and density.

After about 72 hours, when the cell viability drops to below 40%, the cell shake flasks are frozen in a refrigerator at -70 °C or lower and thawed in a water bath at 37 °C for two times. Benzonase (20 U/mL) is added, and reacted in a water bath at 34°C-36°C for 2 hours. The samples are centrifuged at 12000 g for 10 minutes, the supernatant containing viruses is collected, and the precipitate is discarded.

3.2.4 Purification of the recombinant adenovirus

The virus supernatant is adjusted with 5 × equilibration buffer to a conductivity value of 18 mS/cm and pH 7.5. Source 30Q is used to separate and purify the adenovirus particles. The column is equilibrated with solution A (20 mmol/L Tris+150 mmol/L NaCl+2 mmol/L $MgCl_2$, pH7.5), and samples are loaded at a flow rate of 5 mL/min. After the completion of sample loading, solution A is used to equilibrate the column until the UV is decreased to the baseline. Solution B (20 mmol/L Tris+2 mol/L NaCl+2 mmol/L $MgCl_2$, pH7.5) is used for gradient elution at 10 mL/min from 0% to 20% in 50 minutes, and the elution peaks are collected in separate tubes. Finally, 100% solution B is used for elution.

3.3 Identification and titer determination of Ad5-nCoV

3.3.1 PCR amplification of the target gene and gene sequencing

The method and process are the same as those described in section 3.2.1. The results of agarose gel electrophoresis show that a single target band could be amplified in all three vaccine candidates, with correct fragment size. The target bands are extracted and sequenced, and the alignment results indicate that the sequences tested are completely correct.

3.3.2 Infectious unit determination

Clontech Adeno-X™ Rapid Titer Kit is used to measure the infectious unit of the recombinant adenovirus. The procedure is conducted according to the manufacturer's instructions, and the specific method is as follows:

a) HEK293 cells are inoculated into a 24-well plate, with cell density of $5 \times 10^5$ cells/mL, 0.5 mL per well, with MEM + 10% FBS as the medium.
b) The virus to be detected are diluted 10-fold with medium, from $10^{-2}$ to $10^{-6}$, to prepare a series of diluted virus samples, and added to the cells with 50 μL per well.
c) Cells are cultured at 37 °C in 5% $CO_2$ incubator for 48 hours.
d) Cell medium is aspirated and discarded to allow cells to dry slightly (do not over-dry). 0.5 mL of ice-cold methanol is gently added to each well, and left at -20 °C for 10 minutes to fix the cells.
e) Methanol is aspirated and discarded, and cells are gently washed 3 times with PBS+1% BSA. 0.25 mL of Anti-Hexon antibody diluent (1:1000 dilution) is added to each well, and incubates at 37 °C for 1 hour.
f) Anti-Hexon antibody is aspirated and discarded, and cells are gently washed 3 times with PBS+1% BSA. 0.25 mL of HRP-labeled Rat Anti-Mouse Antibody (1:500 dilution) is added to each well, and incubates at 37 °C for 1 hour.
g) Before 0.25 mL of HRP-labeled Rat Anti-Mouse Antibody is aspirated and discarded, 10 × DAB substrate is diluted to 1 × DAB working solution with 1 × Stable Peroxidase Buffer, and allow the solution to reach room temperature.
h) Diluent of Rat Anti-Mouse Antibody is aspirated and discarded, and cells are gently washed 3 times with PBS+1% BSA. 0.25 mL of DAB working solution is added to each well, and left at room temperature for 10 minutes.
i) DAB working solution is aspirated and discarded, and cells are gently washed 2 times with PBS.
j) Brown/black positive cells are counted under a microscope. At least 3 fields are randomly counted for each well, and the mean number of positive cells is calculated.
k) Infectious unit (ifu/mL) is calculated, with the following formula:

$$\text{Infectious unit (ifu/mL)} = \text{Number of positive cells in the field} \times \text{Number of fields in each well/ (Virus volume (mL)} \times \text{Dilution factor)}$$

The results show that after concentration of the purified recombinant adenovirus, the infection titer can reach above $1.0 \times 10^{10}$ ifu / mL.

3.3.3 Determination of the virus particle

Equal volume of 20 mmol/L Tris-Cl, 2 mmol/L EDTA (pH 7.5) and 2.0% SDS solution are mixed to prepare the virus lysate buffer. An appropriate volume of the virus sample to be tested is taken, 1/19 volume of virus lysate buffer is added, the mixture is mixed through repeated blow with pipette for 10 times, and vortexed for 1 minute. The mixture is shaken and digested in a constant temperature water bath at 56 °C for 10 minutes, centrifuged at 12000 rpm for 5 minutes. The supernatant is taken, and the OD values at 260 nm and 280 nm are measured. The number of adenovirus particles is calculated.

Results of the virus particle show that the purified recombinant adenoviruses can reach above $1.0 \times 10^{11}$ VP/mL after concentration.

Example 2. Immunological evaluation of the vaccine candidates in mouse model

1. The humoral immune response induced by the vaccine candidates

100 SPF female BALB/c mice (age 6-8 weeks) are randomly divided into 10 groups, with 10 mice in each group. The mice are immunized with the vaccine candidates based on the grouping as shown in Table 1. For the intramuscular injection, the mouse are injected with 100 μL of the vaccine candidates at the inner side of posterior thigh; for the intranasal immunization, the mouse are anaesthetized with isoflurane, and 20 μL of the vaccine candidates is dripped into the nasal cavity of the mouse. The grouping is shown in Table 1.

Table 1. Grouping of mice tested for humoral immune response induced by the vaccine candidates

| Group | Vaccine candidates | Dose | Route of immunization | No. of mice |
|---|---|---|---|---|
| High-dose | Ad5-nCoV_Sopt | $5\times10^9$ VP | Intramuscular | 10 |
| Medium-dose | Ad5-nCoV_Sopt | $5\times10^8$ VP | Intramuscular | 10 |
| Low-dose | Ad5-nCoV_Sopt | $5\times10^7$ VP | Intramuscular | 10 |
| Control | Ad5 | $1\times10^7$ ifu | Intramuscular | 10 |
| High-dose | Ad5-nCoV_Sopt | $5\times10^9$ VP | Intranasal | 10 |
| Medium-dose | Ad5-nCoV_Sopt | $5\times10^8$ VP | Intranasal | 10 |
| Low-dose | Ad5-nCoV_Sopt | $5\times10^7$ VP | Intranasal | 10 |
| Control | Ad5 | $1\times10^7$ ifu | Intranasal | 10 |
| oriSIP control | Ad5-nCoV _oriSIP-Sopt | $5\times10^8$ VP | Intramuscular | 10 |
| S1 control | Ad5-nCoV_S1opt | $5\times10^8$ VP | Intramuscular | 10 |

Blood samples are collected from the mice at specific time points after immunization, and the serum is separated. The titer of IgG antibody against the S protein of novel coronavirus in the serum is detected by ELISA, and the results is shown in Figures 4-7 (ns, $P\geq0.05$; *, $P<0.05$; **, $P<0.01$; ***, $P<0.001$; ****, $P<0.0001$).

High level of serum IgG antibody can be produced in mice on Day 9 after intramuscular injection with Ad5-nCoV_Sopt, and the mean titer of antibody in the high-dose group is over $10^5$; the level of serum IgG antibody induced by intramuscular injection with Ad5-nCoV_Sopt shows a significant dose-dependent manner, with the higher level of serum IgG antibody response in the higher dose group (see Figure 4). The level of serum antibody furtherly increases from Day 9 to Day 14 after immunization; and on Day 14 after immunization, the titers of serum IgG antibody in the high-dose and low-dose groups are significantly higher than that on Day 9 after immunization (see Figure 5).

The results of serum IgG response induced by intramuscular injection with Ad5-nCoV_Sopt, Ad5-nCoV_oriSIP-Sopt and Ad5-nCoV_S1opt are shown in Figure 6, wherein A indicates the serum IgG antibody titers on Day 9 after immunization; B depicts the serum IgG antibody titers on Day 14 after immunization. The results show that Ad5-nCoV_Sopt induces S protein specific IgG response earlier than the other two candidates. On Day 9 after immunization, the level of serum IgG antibody induced by Ad5-nCoV_Sopt is significantly higher than that of Ad5-nCoV_oriSIP-Sopt and Ad5-nCoV_S1opt (see Figure 6, A). On Day 14 after immunization, the level of serum antibody of Ad5-nCoV_oriSIP-Sopt increases (see Figure 6, B), but the level of serum IgG antibody induced by Ad5-nCoV_S1opt is significantly lower than the other two vaccine candidates both on Day 9 and Day 14 after immunization. The results indicate that among the three vaccine candidates, Ad5-nCoV_Sopt is the fastest to induce the IgG antibody response, with the best immunogenicity.

The serum IgG response induced by intramuscular injection and intranasal immunization with Ad5-nCoV_Sopt on Day 14 after immunization are shown in Figure 7. There is no significant difference in the level of serum IgG antibody induced by the two routes of immunization, regardless of high-dose, medium-dose or low-dose groups.

2. The cellular immune response induced by the vaccine

30 SPF female BALB/c mice aged 6-8 weeks are randomly divided into three groups, with 10 mice in each group. $5\times10^8$ VP of Ad5-nCoV_Sopt is given through intramuscular injection (im.) or intranasal immunization (in.); and $1\times10^7$ ifu Ad5 vector is given through the same immunization routes as the control, with 5 mice per immunization route. For the intramuscular injection, the mouse are injected with 100 $\mu$L of the vaccine candidates at the inner side of posterior thigh; for the intranasal immunization, the mouse are anaesthetized with isoflurane, and 20 $\mu$L of the vaccine candidates is dripped into the nasal cavity of the mouse. The grouping is shown in Table 2.

Table 2. Grouping of cellular immune response testing in Ad5-nCoV_Sopt mouse model

| Group | Antigen | Route of immunization | Dose | No. of animals |
|---|---|---|---|---|
| im. | Ad5-nCoV_Sopt | im. | $5\times10^8$ VP | 10 |
| in. | Ad5-nCoV_Sopt | in. | $5\times10^8$ VP | 10 |
| Control | Ad5 | im. or in. (half and half) | $1\times10^7$ ifu | 10 |

The mice are sacrificed on Day 14 after immunization, and the splenic lymphocytes are separated. The overlapping peptide pool covering the S protein is used for stimulating the lymphocytes for 6 hours in vitro; at the same time, the protein secretion blockers are added to block the secretion of the cytokines. After 6 hours, Fc receptors are blocked, and dead cells and cell surface molecular markers are stained. After the cells are fixed and perforated, intracellular cytokines are stained. Cell surface markers include CD3, CD4, CD8 and CD107a molecules, and intracellular cytokines include IFN$\gamma$, TNF$\alpha$ and IL2. The expression level of IFNy, TNF$\alpha$, IL2 and CD107a in $CD4^+$ T cells and $CD8^+$ T cells after SARS-CoV-2 specific peptides stimulation is determined by Flow cytometry (BD FACS Canto™).

The immune responses of $CD8^+$ T cells and $CD4^+$ T cells induced by Ad5-nCoV_Sopt are shown in Figures 8-9, and representative results are listed in Figures 10-12, wherein Figure 10 indicates the cellular immune response in the intramuscular injection group, Figure 11 depicts the cellular immune response in the intranasal immunization group, and Figure 12 represents the cellular immune response in the control group. The results demonstrate that Ad5-nCoV_Sopt could induce significant cellular immune response in the immunized mice, regardless of intramuscular injection or intranasal immunization group. On Day 14 after immunization, after the splenocytes are stimulated by an overlapping peptide pool covering the SARS-CoV-2 S protein, both $CD8^+$ T cells and $CD4^+$ T cells have significantly higher expression levels of IFNy, TNF$\alpha$ and IL2 than those of the Ad5 vector control group (Control) ($P<0.05$). Meanwhile, compared to intranasal immunization (in.), intramuscular injection (im.) can induce stronger cellular immune response. The levels of IFN$\gamma$, TNF$\alpha$ and IL2 in $CD8^+$ T cells and $CD4^+$ T cells induced by intramuscular injection are significantly higher than those of nasal immunization.

3. The antibody level in trachea-lung washes

Trachea-lung washes is collected when the mice undergoing test for cellular immune response, and the levels of IgG and IgA antibodies against the S1 protein of novel coronavirus in trachea-lung washes are detected by ELISA. Grouping is shown in Table 2, and the results are listed in Figure 13 (****, $P<0.0001$), wherein A indicates the level of IgG antibody in trachea-lung washes; and B represents the level of IgA antibody in trachea-lung washes. The results demonstrate that on Day 14 after immunization with Ad5-nCoV_Sopt, intranasal immunization could induce higher levels of IgG and IgA antibody titers in trachea-lung washes, significantly higher than the intramuscular injection and control groups. In the intramuscular injection group, only low-level IgG antibody titer and no IgA antibody could be detected in trachea-lung washes.

4. Summary of the immunogenicity in mouse model

All the three vaccine candidates, Ad5-nCoV_Sopt, Ad5-nCoV_oriSIP-Sopt and Ad5-nCoV-S1opt, have a good immunogenicity and can induce high levels of serum IgG antibody in mice. Ad5-nCoV_Sopt is the fastest to induce the IgG antibody titer, with the best immunogenicity. Ad5-nCoV_Sopt is selected as the best candidate for recombinant novel coronavirus vaccine, which is marked as Ad5-nCoV. Results of cellular immune response indicate that Ad5-nCoV given by intramuscular injection and intranasal immunization both can induce specific cellular immune responses in the mice model, and intramuscular injection can induce stronger cellular immune responses. Results of the antibody response in trachea-lung washes suggest that intranasal immunization can induce higher levels of IgG and IgA antibodies in trachea-lung washes.

Example 3. Immunological evaluation of Ad5-nCoV in guinea pig model

56 SPF guinea pigs, weighing 200 to 250 g, are randomly divided into 4 groups, with 14 guinea pigs in each group, half male and half female. The guinea pigs are immunized with Ad5-nCoV based on the grouping shown in Table 3. The route of immunization is intramuscular injection with 200 $\mu$L of Ad5-nCoV at the inner side of posterior thigh.

Table 3. Grouping of guinea pigs immunized with Ad5-nCoV

| Group | Vaccine candidates | Dose | Route of immunization | No. of guinea pigs |
|---|---|---|---|---|
| High-dose | Ad5-nCoV | $5\times10^9$ VP | Intramuscularly | 14 |
| Medium-dose | Ad5-nCoV | $5\times10^8$ VP | Intramuscularly | 14 |
| Low-dose | Ad5-nCoV | $5\times10^7$ VP | Intramuscularly | 14 |
| Control | Ad5 | $1\times10^7$ ifu | Intramuscularly | 14 |

Blood samples are collected at specific time after immunization, and serum is separated. The titer of IgG antibody against the S protein of novel coronavirus in the serum is detected by ELISA. As shown in Figure 14 (ns, $P\geq0.05$; *, $P<0.05$; **, $P<0.01$; ***, $P<0.001$; ****, $P<0.0001$), the results indicate that on Day 14 after immunization with Ad5-nCoV, high level of serum IgG antibody titer is detected in guinea pigs. There is no significant difference in antibody titer between the medium-dose group and the high-dose group, but the low-dose group has lower level of serum IgG response, significantly lower than the high-dose and medium -dose groups. The results show that Ad5-nCoV has a good immunogenicity in the guinea pig model.

Example 4. Evaluation of protective effect of Ad5-nCoV on hACE2 transgenic mouse model

Live virus challenge with SARS-CoV-2 (SARS-CoV-2/WH-09/human/2020/ CHN) is conducted in a biosafety level 3 laboratory.

The mice are divided into 3 groups (see Table 4). $5\times10^9$ VP or $5\times10^8$ VP of Ad5-nCoV is injected intramuscularly into the muscle of posterior thigh, with an injection volume of 100 $\mu$L, or the same volume of PBS is injected in the control mice. Two weeks after immunization, the mice are transferred to a biosafety level 3 laboratory for SARS-CoV-2 challenge, with intranasal challenge as the route ($10^5$ $TCID_{50}$/mouse). Observation for 3 consecutive days after challenge is conducted, and weight changes are recorded. All mice are euthanized on Day 3 after infection, and the viral load in lung tissue is tested.

Table 4. Grouping of Ad5-nCoV in animal protection test

| Group | No. of mice | Dose | Challenge | Test indicators |
|---|---|---|---|---|
| High-dose | 6 | $5\times10^9$ VP | $10^5$ $TCID_{50}$/mouse | 3dpi, all mice are sacrificed, viral |
| Low-dose | 6 | $5\times10^8$ VP | $10^5$ $TCID_{50}$/mouse | load in lung tissue and pathological |
| Model | 6 | / | $10^5$ $TCID_{50}$/mouse | examination are performed |

Weight loss after infection is observed in the model group, with the highest mean percentage of weight loss is 3.36%. Compared to the model group, the weight in the high-dose group increases to a certain extent on Day 3 after infection, at 2.55% of the mean percentage of weight increase. The mean weight loss in the low-dose group is 4.72% on Day 3 after infection. Results show that mice in the high-dose group have no obvious symptoms after infection (see Figure 15). As shown in Figure 16, the viral load in lung tissue on Day 3 after infection demonstrates that the viral load in model group is $10^{6.18}$ copies/mL, and $10^{3.11}$ copies/mL in the high-dose group, which is significantly lower than that of the model group ($p<0.001$). The result in the low-dose group is $10^{3.90}$ copies/mL, significantly lower than that of the model group ($p<0.001$). Results indicate that the viral load in lung tissue decreases by 3.07 lg after vaccination with high-dose Ad5-nCoV, and decreases by 2.28 lg after immunization with low-dose Ad5-nCoV. The study results suggest that Ad5-nCoV has a significant protective effect on infected mice.

## INDUSTRIAL APPLICABILITY

The present invention discloses a recombinant 2019 novel coronavirus vaccine for preventing the 2019 novel coronavirus diseases. The said vaccine can be easily industrialized, having the industrial applicability.

## Claims

1. The polynucleotide encoding the S protein of the 2019 novel coronavirus, wherein the sequence of the polynucleotide is shown in SEQ ID NO:1.

2. The vector containing the polynucleotide of Claim 1.

3. The vector of Claim 2, wherein the vector is pDC316.

4. The replication-deficient recombinant human adenovirus expressing the polynucleotide of Claim 1.

5. The recombinant human adenovirus of Claim 4, wherein the recombinant adenovirus is derived from the AdMax adenovirus system.

6. The recombinant human adenovirus of Claims 4 or 5 for use in the preparation of a vaccine for preventing the 2019 novel coronavirus disease.

**7.** The recombinant human adenovirus for use of Claim 6, wherein the recombinant human adenovirus is prepared as an injection, nasal drops or spray.

**8.** The recombinant human adenovirus for use of Claim 7, wherein the recombinant human adenovirus is prepared as an intramuscular injection.

**9.** A method for preparing the recombinant human adenovirus of Claim 4 or 5, wherein the method comprises the following steps:

( 1 ) Construction of a shuttle plasmid vector containing the polynucleotide encoding the 2019 novel coronavirus S protein;
( 2 ) Co-transfection of the shuttle plasmid vector of step (1) and backbone plasmid into a host cell;
( 3 ) Cultivation of the host cells of step (2);
( 4 ) Harvest of the replication-deficient recombinant human adenovirus released from the cells of step (3);
( 5 ) Enlarge cultivation of the recombinant human adenovirus of step (4);
( 6 ) Purification of the culture product of step (5).

**10.** The method of Claim 9, wherein the vector of step (1) is pDC316.

**11.** The method of Claim 9, wherein the backbone plasmid of step (2) is pBHGlox∆E1,3Cre.

**12.** The method of Claim 9, wherein the cell of step (3) is HEK293 cells.

**13.** The method of Claim 9, wherein the enlarge culture method of step (5) is suspension culture.

**14.** The method of Claim 9, wherein the purification method of step (6) is Source 30 Q chromatography.

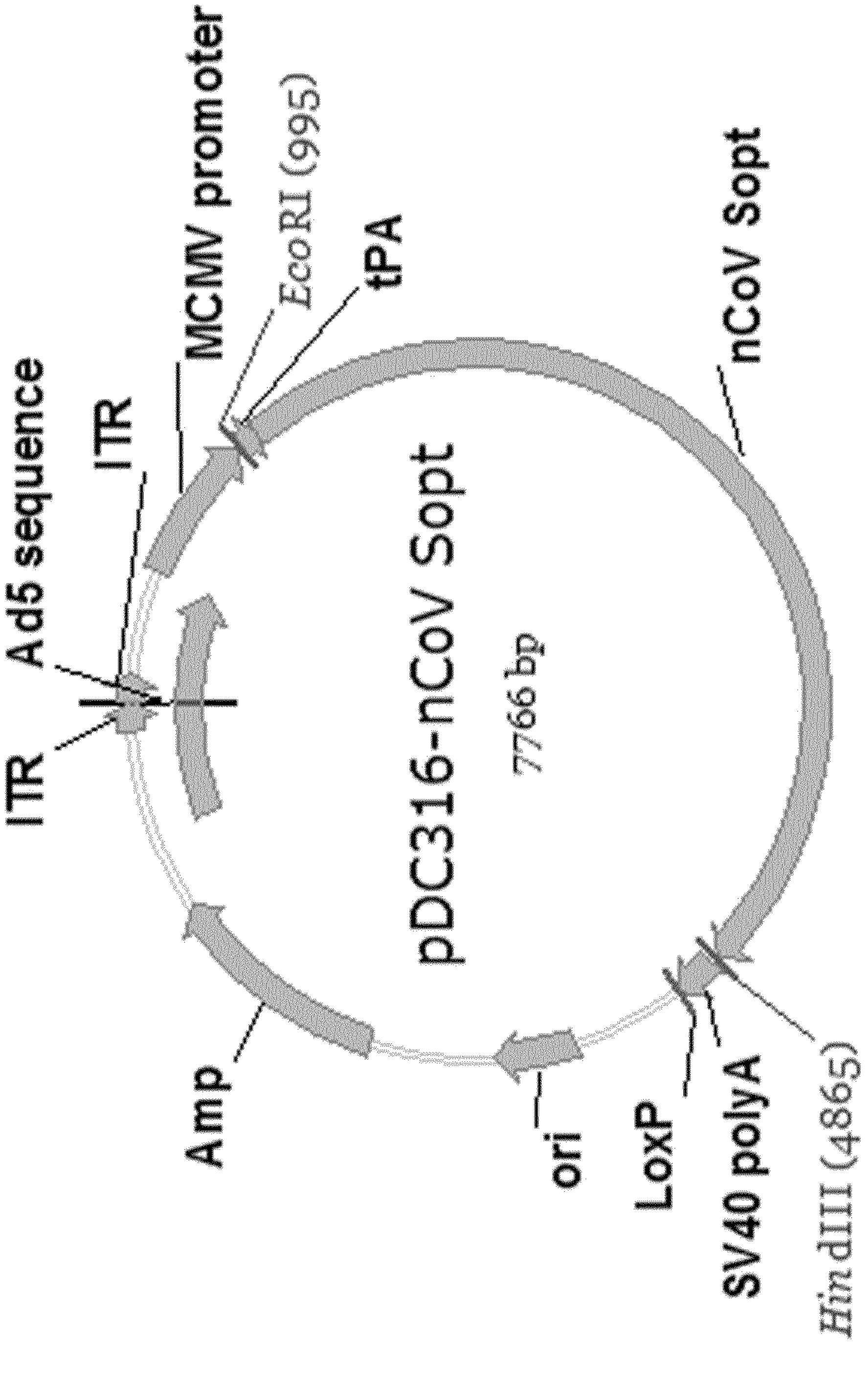
ITR
Ad5 sequence
ITR
MCMV promoter
EcoRI (995)
tPA
nCoV Sopt
pDC316-nCoV Sopt
7766 bp
Amp
ori
LoxP
SV40 polyA
HindIII (4865)

FIG 1

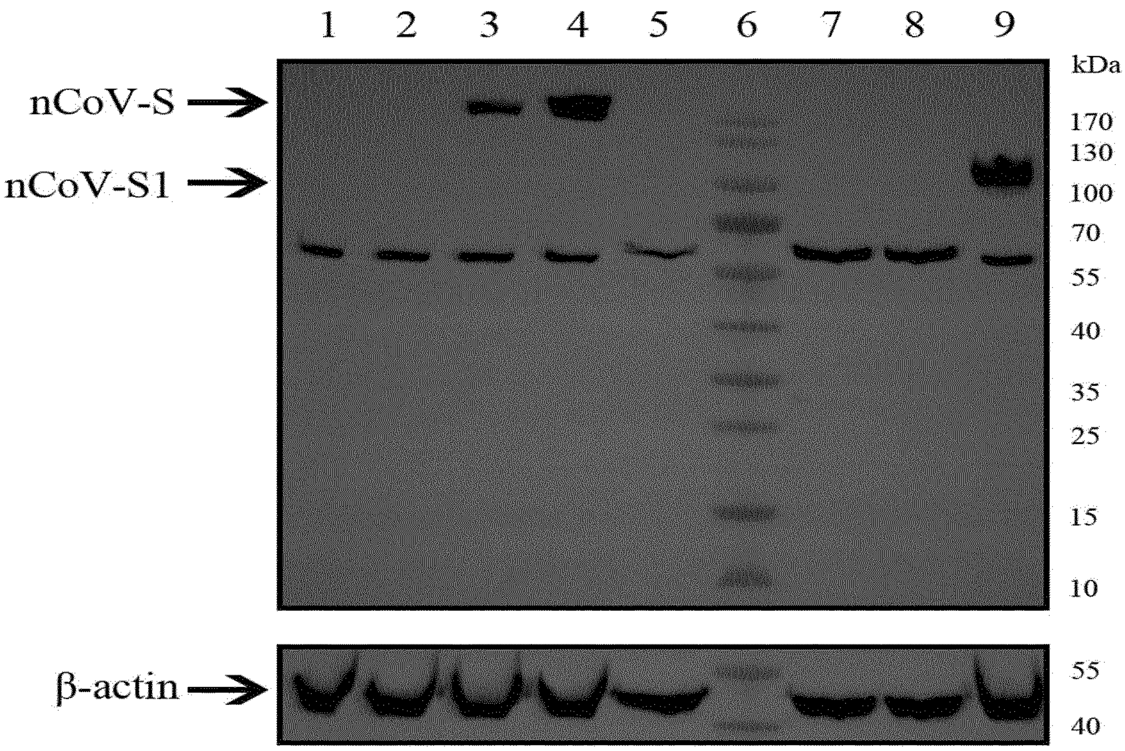
1
2
3
4
5
6
7
8
9
kDa
nCoV-S
nCoV-S1
170
130
100
70
55
40
35
25
15
10
β-actin
55
40

FIG 2

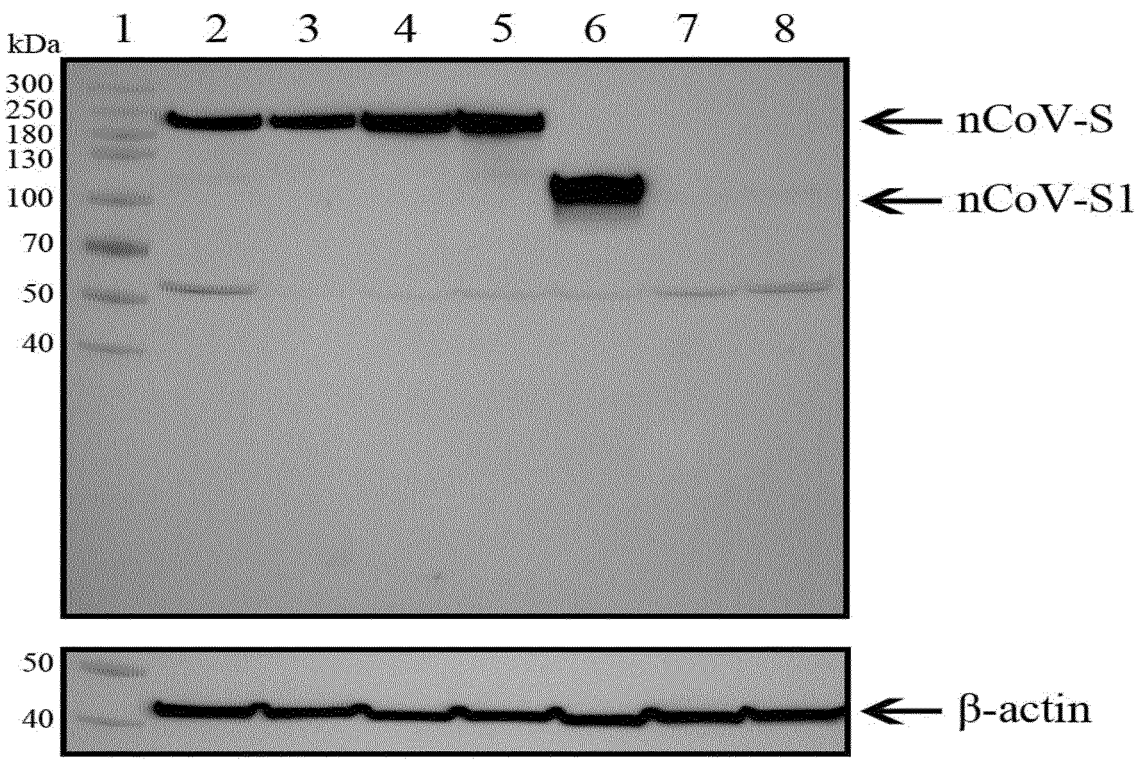
kDa
1
2
3
4
5
6
7
8
300
250
180
130
100
70
50
40
nCoV-S
nCoV-S1
50
40
β-actin

FIG 3

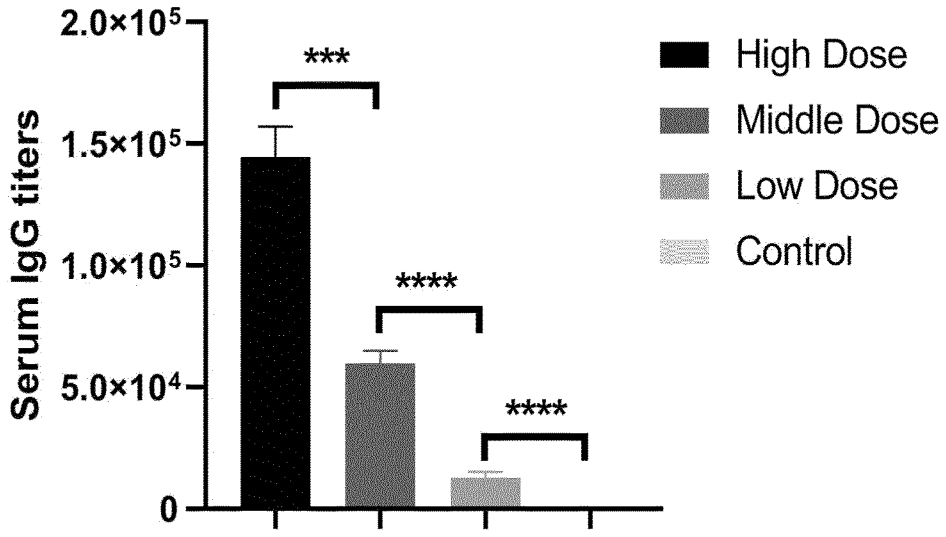
2.0×10⁵
1.5×10⁵
1.0×10⁵
5.0×10⁴
0
Serum IgG titers
***
****
****
High Dose
Middle Dose
Low Dose
Control

FIG 4

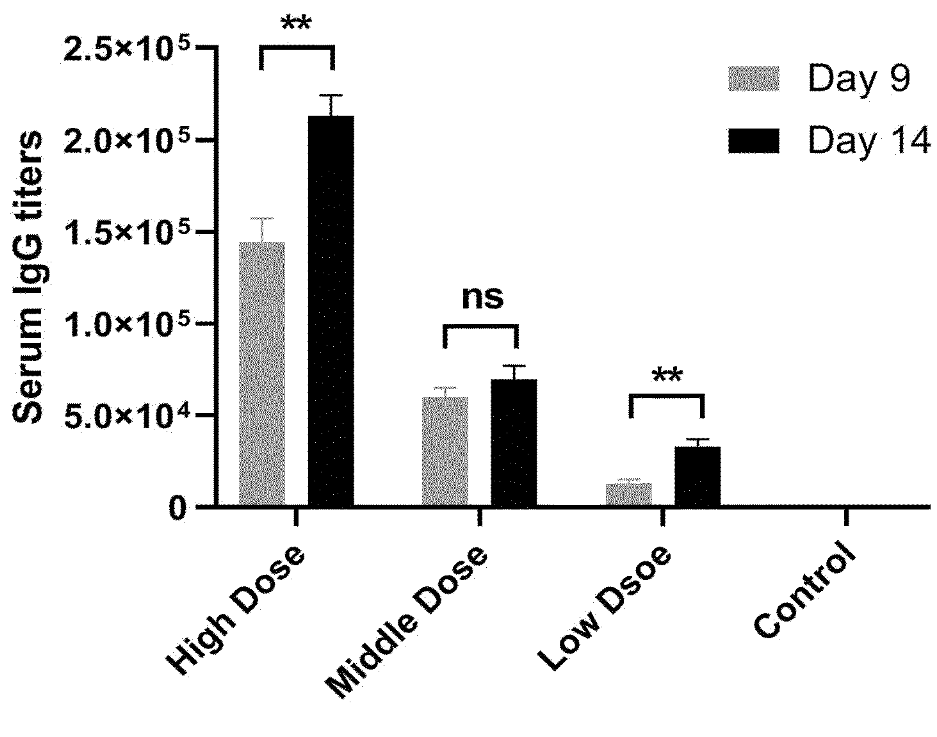
2.5×10⁵
2.0×10⁵
1.5×10⁵
1.0×10⁵
5.0×10⁴
0
Serum IgG titers
**
ns
**
Day 9
Day 14
High Dose
Middle Dose
Low Dsoe
Control

FIG 5

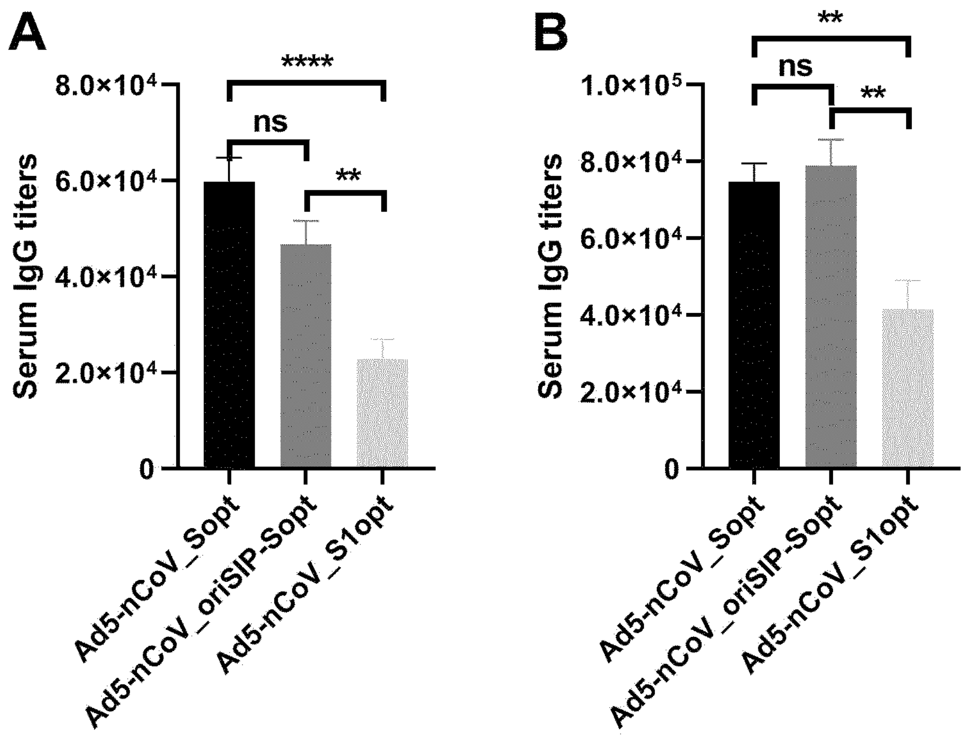
A
B
Serum IgG titers
8.0×10⁴
6.0×10⁴
4.0×10⁴
2.0×10⁴
0
****
ns
**
Ad5-nCoV_Sopt
Ad5-nCoV_oriSIP-Sopt
Ad5-nCoV_S1opt
1.0×10⁵
8.0×10⁴
6.0×10⁴
4.0×10⁴
2.0×10⁴
0
**
ns
**
Ad5-nCoV_Sopt
Ad5-nCoV_oriSIP-Sopt
Ad5-nCoV_S1opt

FIG 6

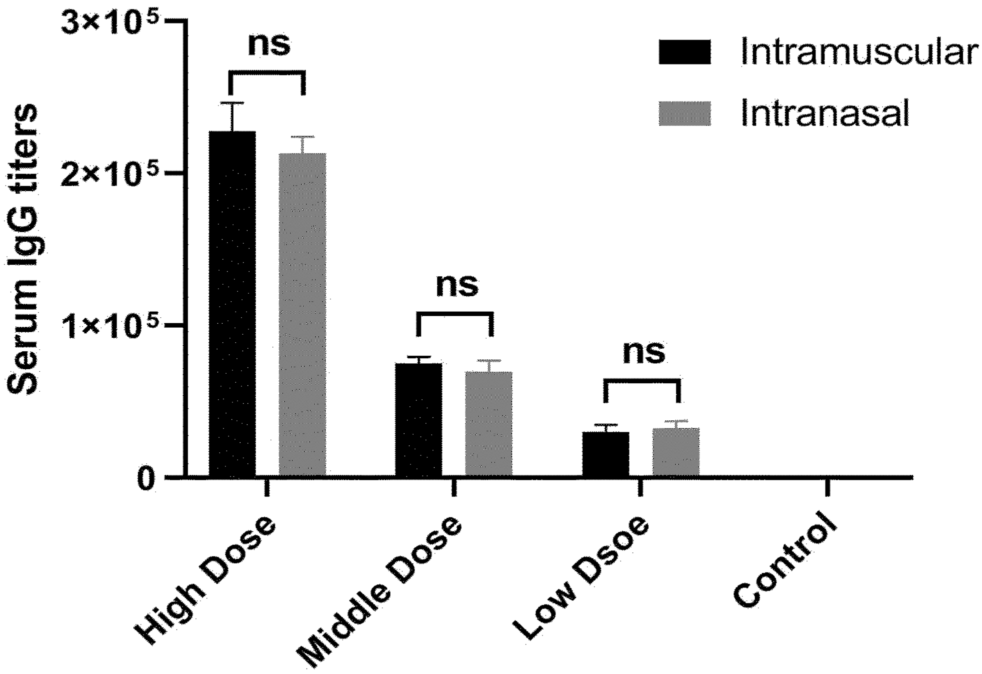
Serum IgG titers
3×10⁵
2×10⁵
1×10⁵
0
Intramuscular
Intranasal
ns
ns
ns
High Dose
Middle Dose
Low Dsoe
Control

FIG 7

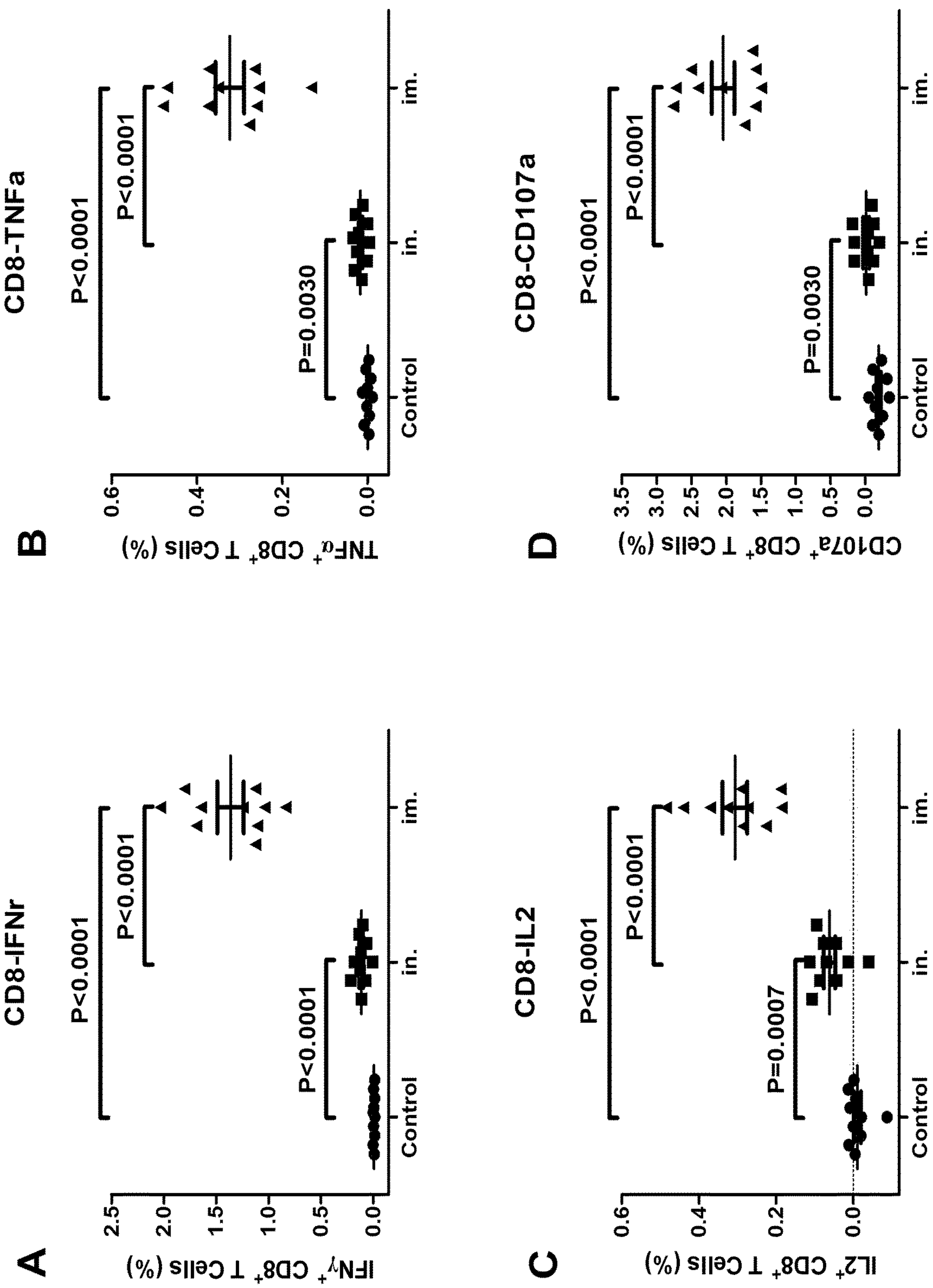
A
CD8-IFNr
P<0.0001
P<0.0001
P<0.0001
IFNγ+ CD8+ T Cells (%)
Control
in.
im.
B
CD8-TNFa
P<0.0001
P<0.0001
P=0.0030
TNFα+ CD8+ T Cells (%)
Control
in.
im.
C
CD8-IL2
P<0.0001
P<0.0001
P=0.0007
IL2+ CD8+ T Cells (%)
Control
in.
im.
D
CD8-CD107a
P<0.0001
P<0.0001
P=0.0030
CD107a+ CD8+ T Cells (%)
Control
in.
im.

FIG 8

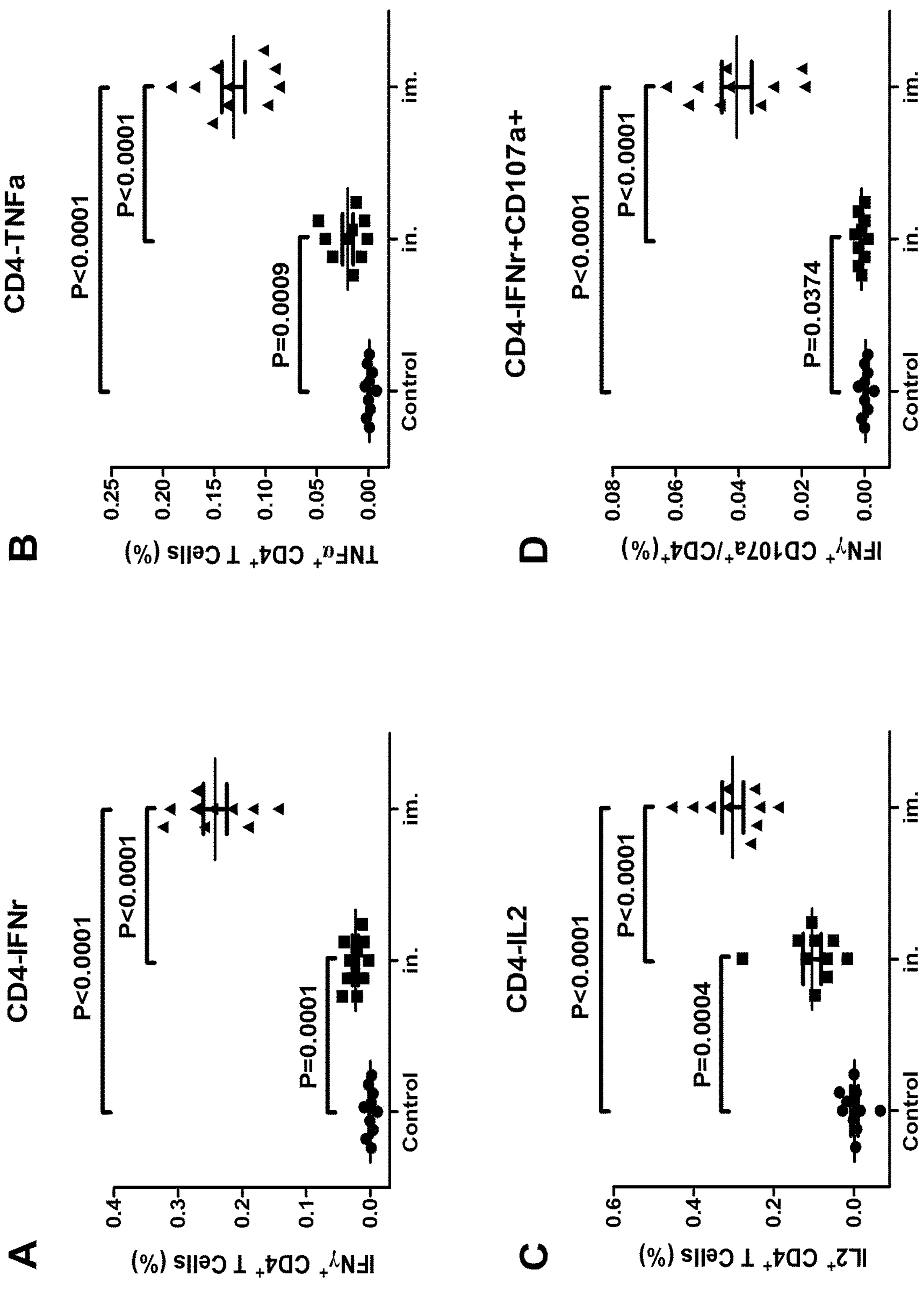
A
CD4-IFNr
IFNγ+ CD4+ T Cells (%)
P<0.0001
P<0.0001
P=0.0001
Control
in.
im.
B
CD4-TNFa
TNFα+ CD4+ T Cells (%)
P<0.0001
P<0.0001
P=0.0009
C
CD4-IL2
IL2+ CD4+ T Cells (%)
P<0.0001
P<0.0001
P=0.0004
D
CD4-IFNr+CD107a+
IFNγ+ CD107a+/CD4+(%)
P<0.0001
P<0.0001
P=0.0374

FIG 9

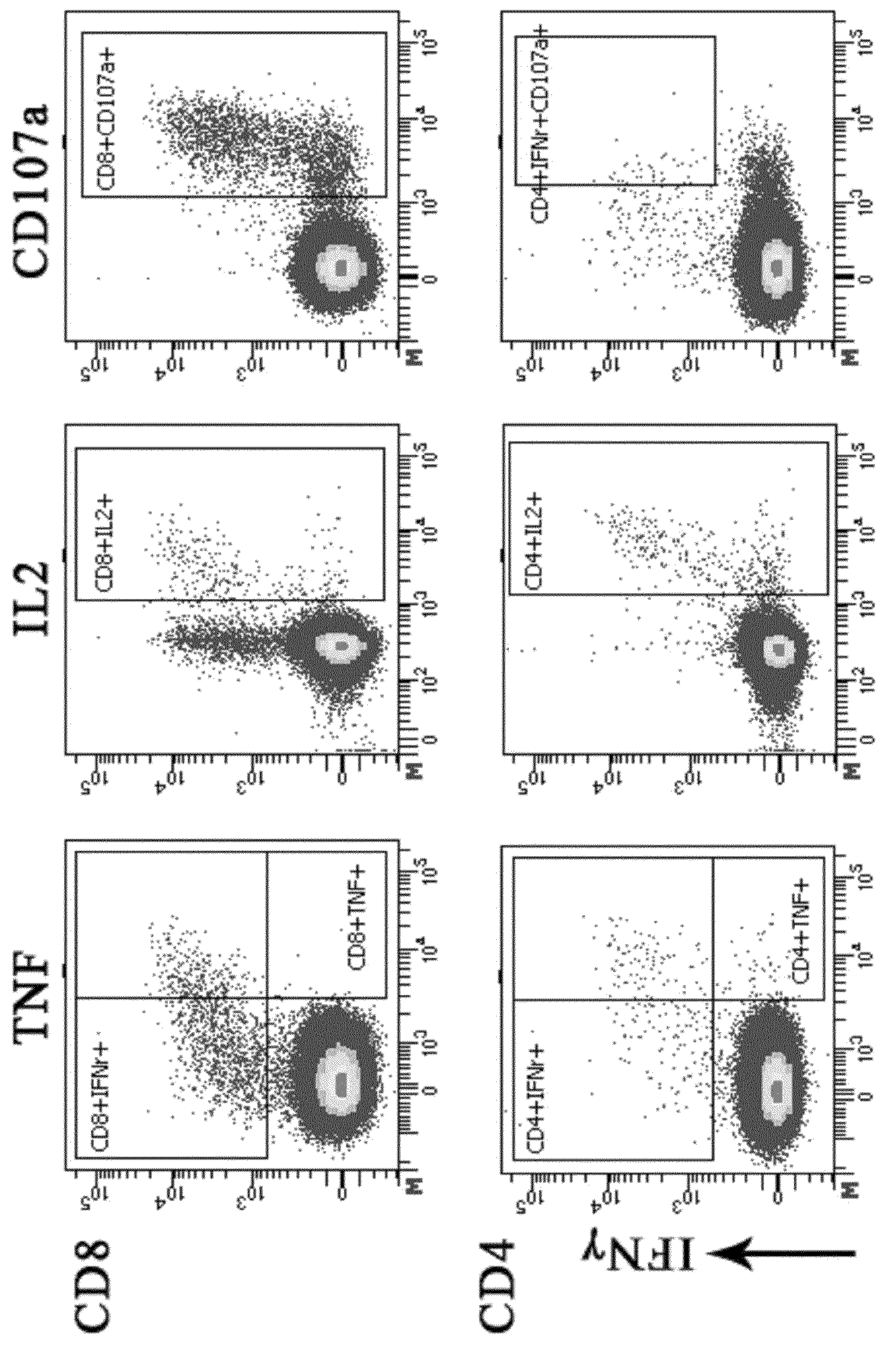
CD107a
IL2
TNF
CD8
CD4
IFNγ
CD8+CD107a+
CD4+IFNr+CD107a+
CD8+IL2+
CD4+IL2+
CD8+IFNr+
CD8+TNF+
CD4+IFNr+
CD4+TNF+

FIG 10

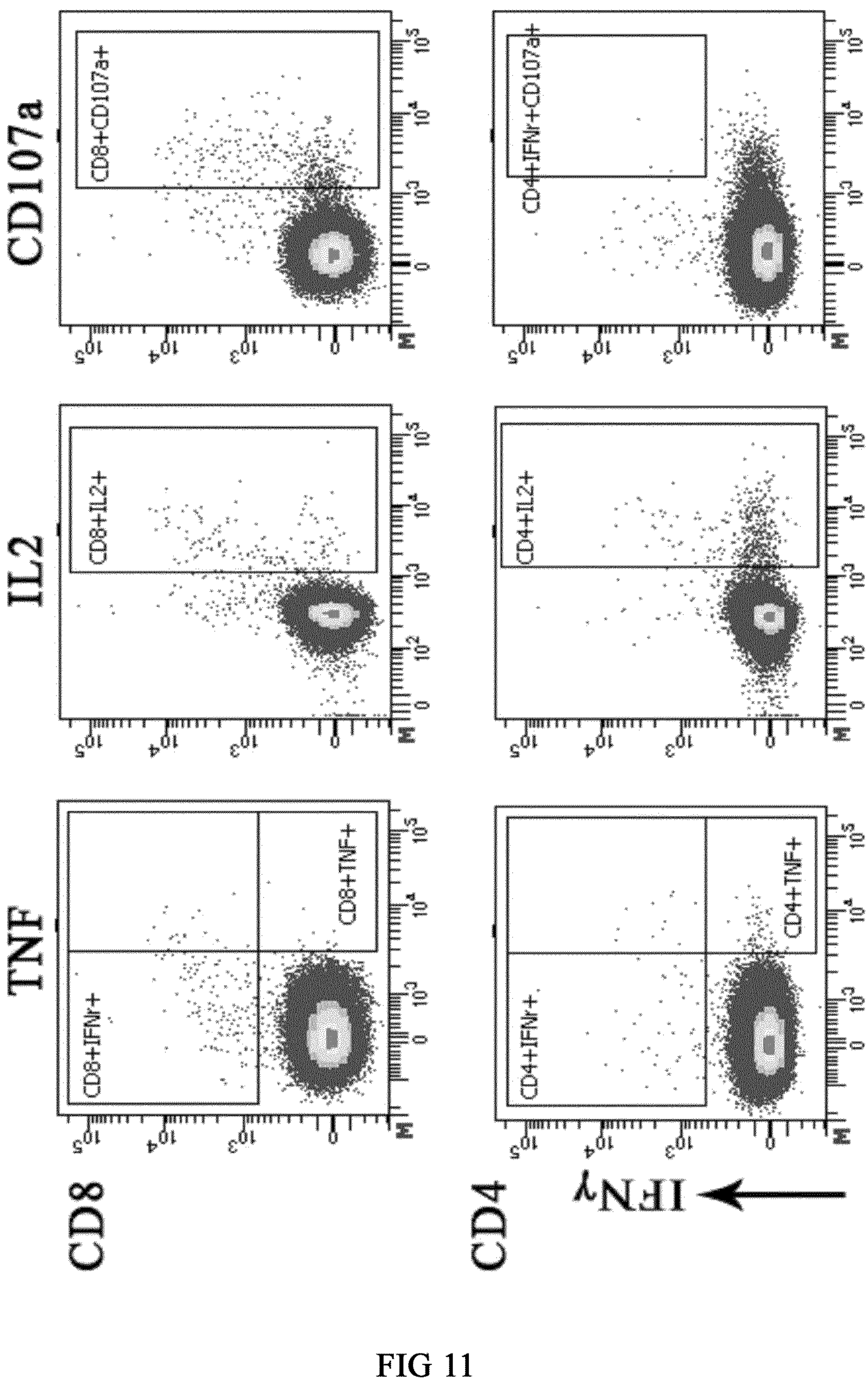
TNF
IL2
CD107a
CD8
CD4
IFNγ
CD8+IFNr+
CD8+TNF+
CD8+IL2+
CD8+CD107a+
CD4+IFNr+
CD4+TNF+
CD4+IL2+
CD4+IFNr+CD107a+

FIG 11

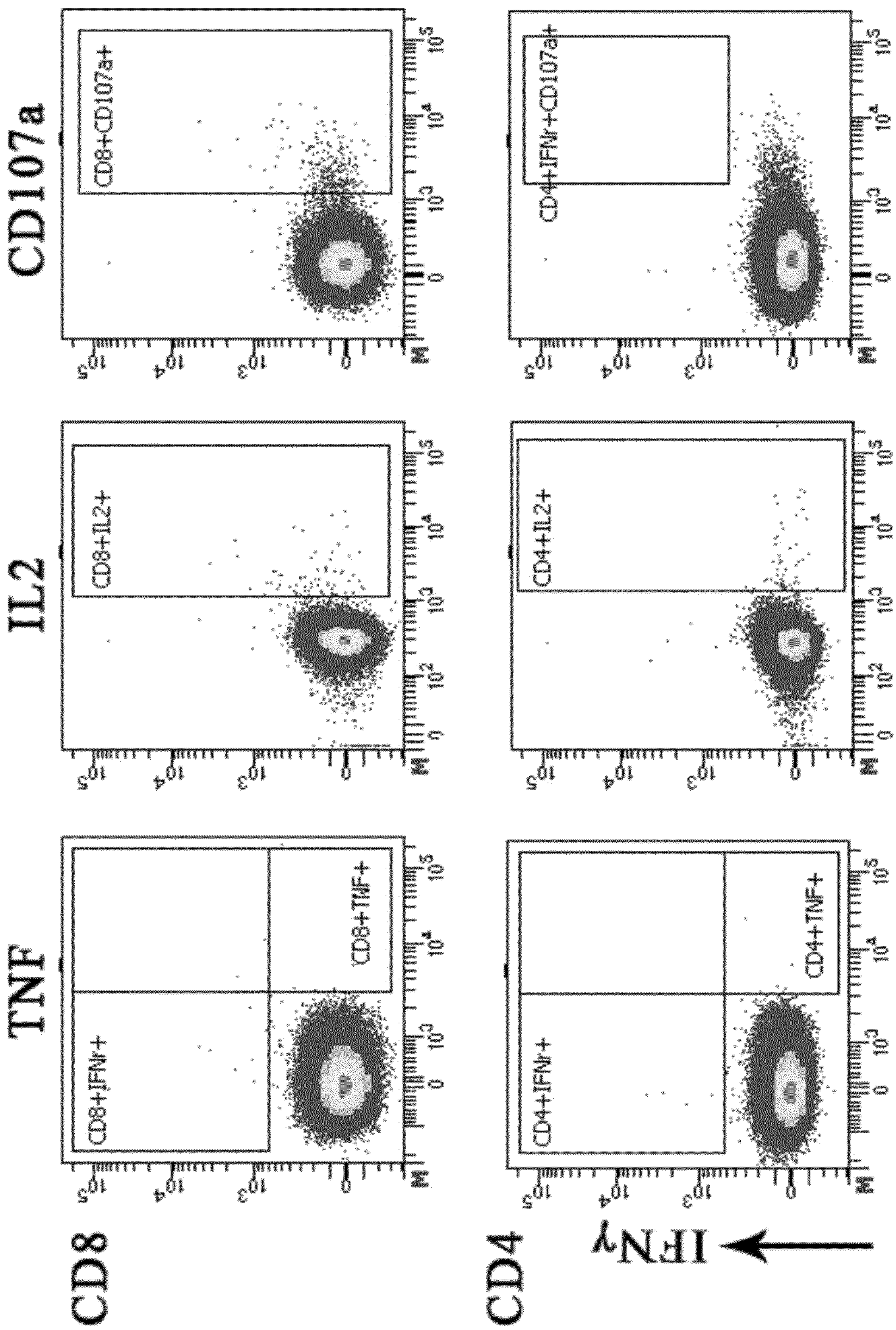
CD107a
IL2
TNF
CD8
CD4
IFNγ
CD8+CD107a+
CD4+IFNr+CD107a+
CD8+IL2+
CD4+IL2+
CD8+IFNr+
CD8+TNF+
CD4+IFNr+
CD4+TNF+

FIG 12

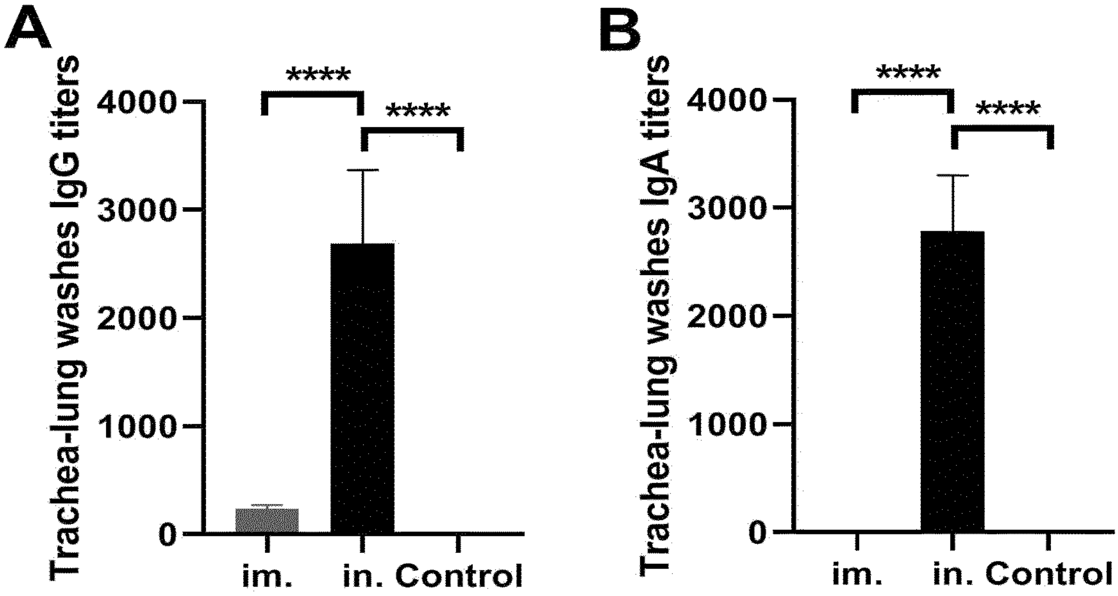
A
B
****
****
Trachea-lung washes IgG titers
Trachea-lung washes IgA titers
4000
3000
2000
1000
0
im.
in.
Control

FIG 13

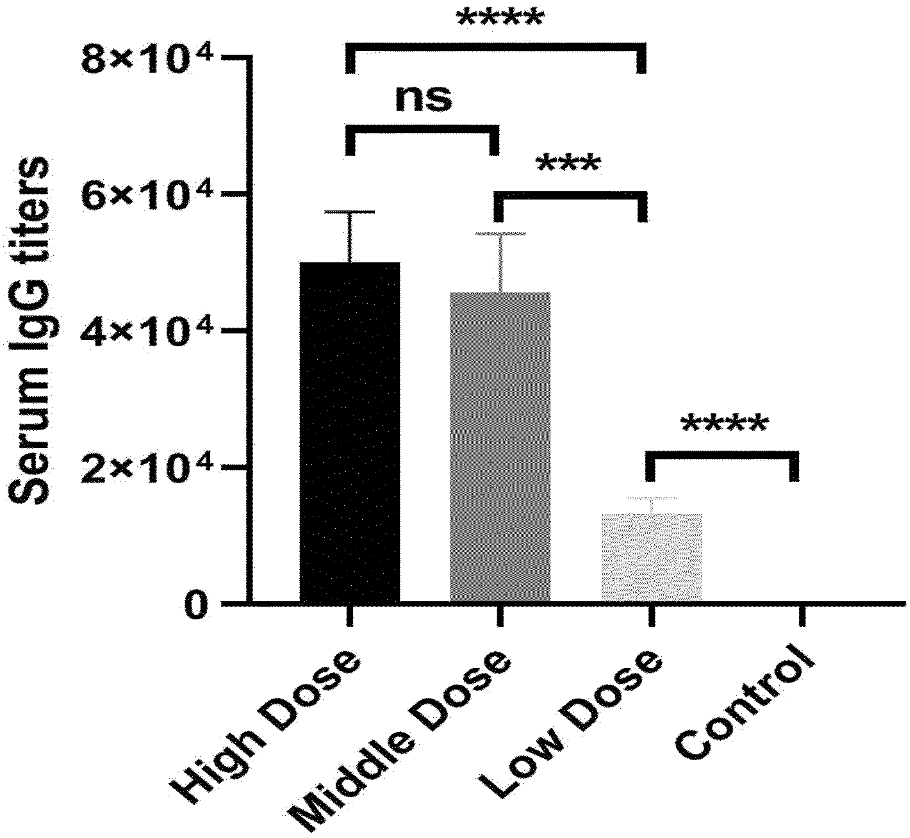
****
ns
***
****
Serum IgG titers
8×10⁴
6×10⁴
4×10⁴
2×10⁴
0
High Dose
Middle Dose
Low Dose
Control

FIG 14

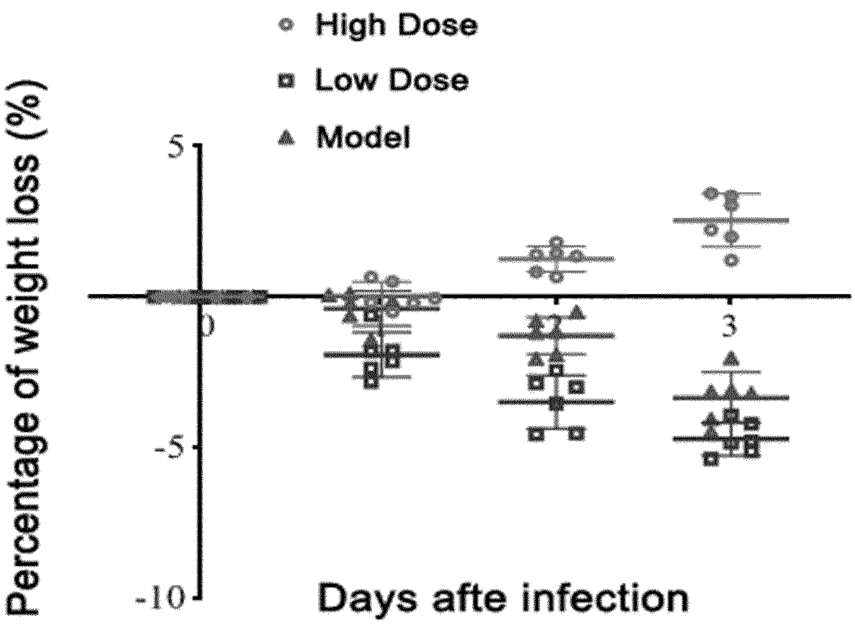
High Dose
Low Dose
Model
5
0
-5
-10
2
3
Percentage of weight loss (%)
Days afte infection

FIG 15

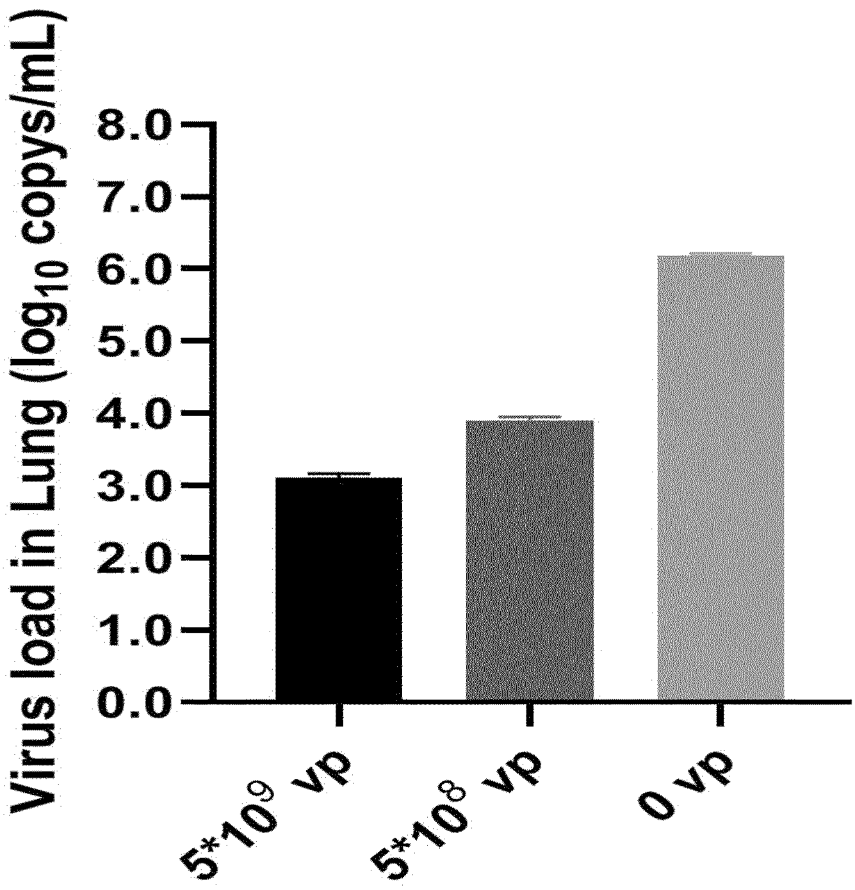
Virus load in Lung ($log_{10}$ copys/mL)
8.0
7.0
6.0
5.0
4.0
3.0
2.0
1.0
0.0
5*10$^9$ vp
5*10$^8$ vp
0 vp

FIG 16

**INTERNATIONAL SEARCH REPORT**

International application No. **PCT/CN2020/096024**

**A. CLASSIFICATION OF SUBJECT MATTER**

C12N 15/50(2006.01)i; C07K 14/165(2006.01)i; C12N 15/861(2006.01)i; A61K 39/215(2006.01)i; A61P 31/14(2006.01)i

According to International Patent Classification (IPC) or to both national classification and IPC

**B. FIELDS SEARCHED**

Minimum documentation searched (classification system followed by classification symbols)

C12N; C07K; A61K; A61P

Documentation searched other than minimum documentation to the extent that such documents are included in the fields searched

Electronic data base consulted during the international search (name of data base and, where practicable, search terms used)

CNABS; SIPOABS; DWPI; CNTXT; WOTXT; USTXT; EPTXT; ISI WEB OF SCIENCE; PUBMED; CNKI; 万方数据库: 新型冠状病毒, 新冠病毒, S 蛋白, 刺突蛋白, 疫苗, 腺病毒, SARS-CoV-2, 2019-nCoV, new coronavirus, S protein, spike protein, vaccine, adenovirus, tPA; 中国专利生物序列检索系统+GenBank+EMBL: sequence search for SEQ ID NOs: 1 and 2.

**C. DOCUMENTS CONSIDERED TO BE RELEVANT**

| Category* | Citation of document, with indication, where appropriate, of the relevant passages | Relevant to claim No. |
|---|---|---|
| PX | CN 111218459 A (INSTITUTE OF MILITARY MEDICINE, ACADEMY OF MILITARY SCIENCES OF PLA et al.) 02 June 2020 (2020-06-02)<br>see claims 1-14 | 1-14 |
| A | CN 1791427 A (CHEN, Yiyou) 21 June 2006 (2006-06-21)<br>see entire document | 1-14 |
| A | "NCBI see sequence : NC_045512.2"<br>*GenBANK,* 13 March 2020 (2020-03-13),<br>see sequence and related information thereof | 1-14 |
| A | 张明顺 等 (ZHANG, Mingshun et al.). "冠状病毒疫苗研究进展及2019新型冠状病毒疫苗研发展望 (Research and Development of Coronavirus Vaccine and Prospect of 2019 Novel Coronavirus Vaccines)"<br>*南京医科大学学报 (自然科学版) (Acta Universitatis Medicinalis Nanjing ((Natural Science)),* Vol. 40, No. 2, 28 February 2020 (2020-02-28),<br>ISSN: 1007-4368,<br>pp. 151-154, see entire document | 1-14 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 November 2020** | **17 December 2020** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/096024**

**C. DOCUMENTS CONSIDERED TO BE RELEVANT**

| Category* | Citation of document, with indication, where appropriate, of the relevant passages | Relevant to claim No. |
|---|---|---|
| A | 陈嘉源 等 (CHEN, Jiayuan et al.). "2019 新型冠状病毒基因组的生物信息学分析(网络首发) (Bioinformatics Analysis of the 2019 Novel Coronavirus Genome, Advance Online Publications)" *生物信息学 (Chinese Journal of Bioinformatics),* 21 January 2020 (2020-01-21), ISSN: 1672-5565, pp. 1-11, see entire document | 1-14 |
| A | 刘彬 等 (LIU, Bin et al.). "新型冠状病毒基因组结构与蛋白功能 (Genomic Structure and Protein Profiles of 2019 Novel Coronavirus)" *微生物与感染 (Journal of Microbes and Infections),* Vol. 1, No. 15, 25 February 2020 (2020-02-25), ISSN: 1673-6184, pp. 52-57, see entire document | 1-14 |

Form PCT/ISA/210 (second sheet) (January 2015)

**INTERNATIONAL SEARCH REPORT**

International application No.
**PCT/CN2020/096024**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

**INTERNATIONAL SEARCH REPORT**
**Information on patent family members**

International application No.
**PCT/CN2020/096024**

| Patent document cited in search report | | | Publication date (day/month/year) | Patent family member(s) | Publication date (day/month/year) |
|---|---|---|---|---|---|
| CN | 111218459 | A | 02 June 2020 | None | |
| CN | 1791427 | A | 21 June 2006 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GAO, W. RZEWSKI ; A. SUN ; H. ROBBINS ; P. D. &GAMBOTTO ; A. UPGENE.** Application of a web-based DNA codon optimization algorithm. *Biotechnol Prog,* 2004, vol. 20 (2), 443-8